# EUROPÄISCHE PATENTSCHRIFT

(11) **EP 3 029 045 B1**
(45) Veröffentlichungstag und Bekanntmachung des Hinweises auf die Patenterteilung: **13.06.2018**
(21) Anmeldenummer: 14196179.7
(22) Anmeldetag: 04.12.2014
(51) Int. Cl.: B01J 31/18, C07F 9/6571, C07F 15/00, C07F 9/6574, C07B 41/06, C07C 45/50

(54) **Bisphosphite die eine 2,3 -Biphenol-Einheit als Zentral-Baustein aufweisen**
Bis-phosphites with 2.3 -biphenol unit as central component
Bisphosphites présentant une unité de 2,3-bisphénol en tant que composant central

(43) Veröffentlichungstag der Anmeldung: 08.06.2016
(73) Patentinhaber: Evonik Degussa GmbH, 45128 Essen (DE)
(72) Erfinder: Dyballa, Katrin Marie, 45657 Recklinghausen (DE); Franke, Robert, 45772 Marl (DE); Börner, Armin, 18059 Rostock (DE); Selent, Detlef, 18059 Rostock (DE)

(56) Entgegenhaltungen:
- WO-A1-2008/071508
- WO-A1-2010/030339
- WO-A1-2011/046781
- ROBERT FRANKE ET AL: "Applied Hydroformylation", CHEMICAL REVIEWS, Bd. 112, Nr. 11, 14. November 2012 (2012-11-14), Seiten 5675-5732, XP055091930, ISSN: 0009-2665, DOI: 10.1021/cr3001803

## Beschreibung

Die Erfindung betrifft Bisphosphite, die eine 2,3'-Biphenol-Einheit als Zentral-Baustein aufweisen. Des Weiteren deren Verwendung als Liganden in der Hydroformylierung.

Ein Bisphosphit weist einen Zentral-Baustein, das so genannte Rückgrat, und zwei Flügel-Bausteine auf, welche mit dem Zentral-Baustein über das P-Atom verbunden sind.

Die erfindungsgemäßen Verbindungen weisen als Zentral-Baustein eine 2,3'-Biphenol-Einheit auf.

Unter 2,3'-Biphenol-Einheit ist eine Biphenol-Einheit zu verstehen, welche die folgende Struktur aufweist:

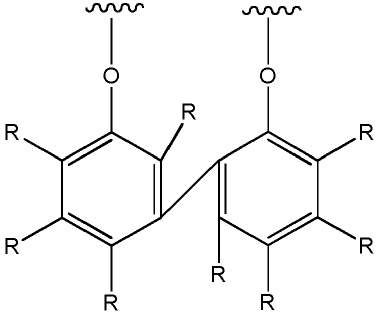

Die Nummerierung der C-Atome in den Ringen kann im IUPAC-Namen der Verbindung, je nach zusätzlichen Substituenten, zu einer anderen Positionsangabe als 2 und 3 führen.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigen Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd)) erreicht. In WO 95/30680 werden zweizähnige Phosphinliganden und ihr Einsatz in der Katalyse, unter anderem auch in Hydroformylierungsreaktionen, offenbart.

Weitere Bisphosphitliganden auf Basis eines 2,2'-verknüpften Biphenol-Bausteins, sowie die Verwendung solcher Bisphosphite als Liganden in Hydroformylierungskatalysatoren sind bekannt aus WO2010/030339 (siehe "Biphephos" dort), WO2011/046781 und WO2008/071508. Die meisten im Stand der Technik bekannten Bisphosphite zeichnen sich dadurch aus, dass diese ein 2,2'-Biphenol als Zentralbaustein, also im Rückgrat, haben. Die Verwendung eines 2,3'-Biphenols als Zentralbaustein ist gänzlich unbekannt.

Obgleich eine Vielzahl von Liganden und ihre Verwendung in der Rhodium-katalysierten Hydroformylierung bekannt sind, ist es wünschenswert, neue Liganden mit verbesserten Eigenschaften zu entwickeln.

Der Erfindung lag die Aufgabe zugrunde, Bisphosphite bereitzustellen, welche gegenüber den bekannten Bisphosphiten vorteilhafte Eigenschaften in der Hydroformylierung aufweisen. Insbesondere bestand die Aufgabe darin, neue Liganden bereitzustellen, die neben einer guten Ausbeute auch noch eine hohe n-Selektivität der korrespondierende Aldehyde bei der Umsetzung von endständigen Olefinen generieren und die ebenfalls bei der Hydroformylierung von innenständigen Olefinen zufriedenstellende n/i-Selektivitäten aufweisen. Es soll also neben einer sehr guten Ausbeute auch noch zusätzlich eine gute Selektivität erzielt werden.

Insbesondere sollen die Ausbeute und/oder die Selektivität gesteigert werden.

Die Aufgabe wird gelöst durch eine Verbindung nach Anspruch 1.

Verbindung, welche eine der beiden allgemeinen Strukturen **I** oder **II** aufweist: 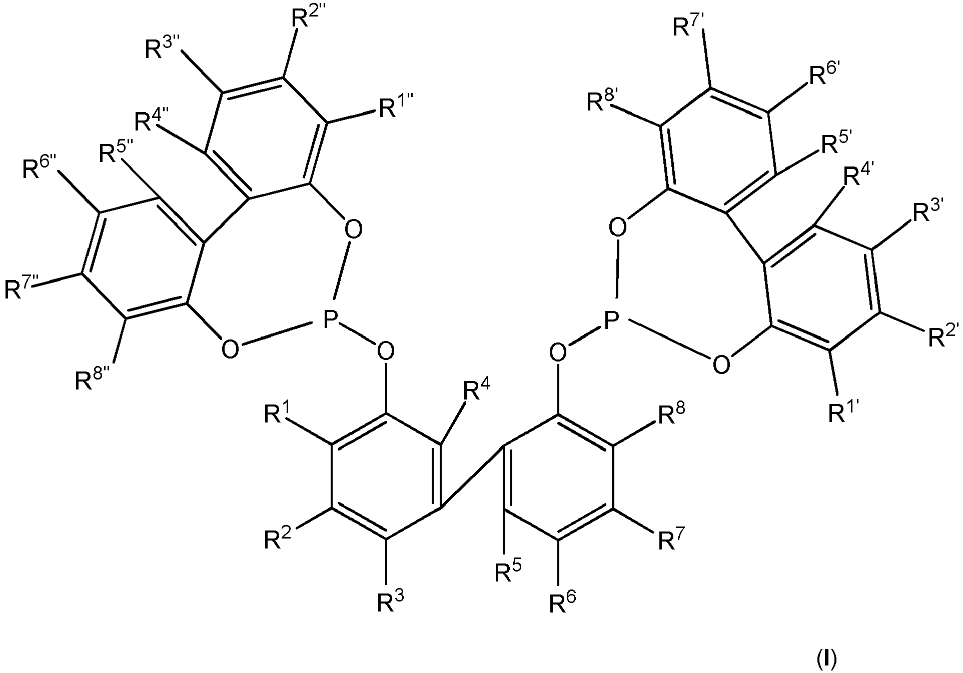 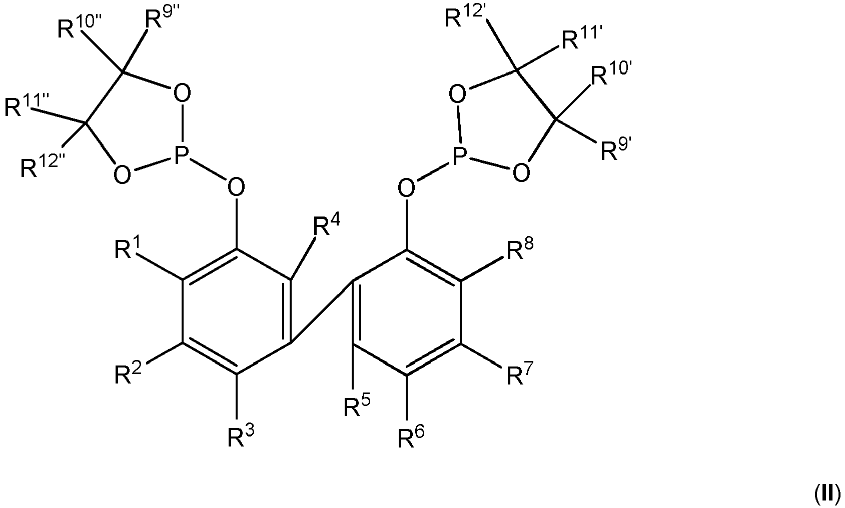 wobei
R¹, R², R³, R⁴, R⁵, R⁶, R⁷, R⁸ ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹', R²', R³', R⁴', R⁵', R⁶', R⁷', R⁸', R¹", R²" , R³" , R⁴" , R⁵" , R⁶" , R⁷" , R⁸" ausgewählt sind aus:
   -H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH,-SO₃H, -NH₂, -N[(C₁-C₁₂)-Alkyl]₂;
R⁹', R¹⁰', R¹¹', R¹²', R⁹", R¹⁰", R¹¹", R¹²" ausgewählt sind aus:
   -H, -(C₆-C₂₀)-Aryl;
wobei die genannten Alkyl- und Arylgruppen substituiert sein können.

(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl. Substituierte (C6-C20)-Aryl und -(C₆-C₂₀)-Aryl-(C₆-C₂₀)-Aryl- können, in Abhängigkeit von der Ringgrösse, einen oder mehrere Substituenten aufweisen; diese Substituenten sind unabhängig voneinander ausgewählt unter: -H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I), -COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂.

Im Rahmen der Erfindung umfasst der Ausdruck -(C₁-C₁₂)-Alkyl geradkettige und verzweigte Alkylgruppen. Vorzugsweise handelt es sich dabei um unsubstituierte geradkettige oder verzweigte -(C₁-C₈)-Alkyl- und ganz bevorzugt -(C₁-C₆)-Alkylgruppen. Beispiele für -(C₁-C₁₂)-Alkylgruppen sind insbesondere Methyl, Ethyl, Propyl, Isopropyl, n-Butyl, iso-Butyl, sec.-Butyl, tert.-Butyl, n-Pentyl, 2-Pentyl, 2-Methylbutyl-, 3-Methylbutyl-, 1,2-Dimethylpropyl-, 1,1-Dimethylpropyl, 2,2-Dimethylpropyl-, 1-Ethylpropyl-, n-Hexyl-, 2-Hexyl-, 2-Methylpentyl-, 3-Methylpentyl-, 4-Methylpentyl-, 1,1-Dimethylbutyl-, 1,2-Diemthylbutyl-, 2,2-Dimethylbutyl-, 1,3-Dimethylbutyl-, 2,3-Dimethylbutyl-, 3,3-Dimethylbutyl-, 1,1,2-Trimethylpropyl-, 1,2,2-Trimethylpropyl-, 1-Ethylbutyl-, 1-Ethyl-2-methylpropyl-, n-Heptyl-, 2-Heptyl-, 3-Heptyl-, 2-Ethylpentyl-, 1-Propylbutyl-, n-Octyl-, 2-Ethylhexyl-, 2-Propylheptyl-, Nonyl-, Decyl.

Die Erläuterungen zum Ausdruck -(C₁-C₁₂)-Alkyl gelten auch für die Alkylgruppen in -O-(C₁-C₁₂)-Alkyl, also in -(C₁-C₁₂)-Alkoxy. Vorzugsweise handelt es sich dabei um unsubstituierte geradkettige oder verzweigte -(C₁-C₆)-Alkoxygruppen.

Substituierte -(C₁-C₁₂)-Alkylgruppen und substituierte -(C₁-C₁₂)-Alkoxygruppen können in Abhängigkeit von ihrer Kettenlänge, einen oder mehrere Substituenten aufweisen. Die Substituenten sind vorzugsweise unabhängig voneinander ausgewählt unter -(C₃-C₁₂)-Cycloalkyl, -(C₃-C₁₂)-Heterocycloalkyl, -(C₆-C₂₀)-Aryl, Fluor, Chlor, Cyano, Formyl, Acyl oder Alkoxycarbonyl.

Der Ausdruck -(C₃-C₁₂)-Cycloalkyl umfasst im Sinne der vorliegenden Erfindung mono-, bi- oder tricyclische Kohlenwasserstoffreste mit 3 bis 12, insbesondere 5 bis 12 Kohlenstoffatomen. Dazu zählen Cyclopropyl-, Cyclobutyl-, Cyclopentyl-, Cyclohexyl-, Cycloheptyl-, Cyclooctyl-, Cyclododecyl-, Cyclopentadecyl-, Norbonyl- oder Adamantyl. Ein Beispiel für ein substituiertes Cycloalkyl wäre Menthyl.

Der Ausdruck -(C₃-C₁₂)-Heterocycloalkylgruppen umfasst im Sinne der vorliegenden Erfindung nichtaromatische, gesättigte oder teilweise ungesättigte cycloaliphatische Gruppen mit 3 bis 12, insbesondere 5 bis 12, Kohlenstoffatomen. Die -(C₃-C₁₂)-Heterocycloalkylgruppen weisen vorzugsweise 3 bis 8, besonders bevorzugt 5 oder 6, Ringatome auf. In den Heterocycloalkylgruppen sind im Unterschied zu den Cycloalkylgruppen 1, 2, 3 oder 4 der Ringkohlenstoffatome durch Heteroatome oder heteroatomhaltige Gruppen ersetzt. Die Heteroatome oder die heteroatomhaltige Gruppen sind vorzugsweise ausgewählt unter -O-, -S-, -N-, -N(=O)-, -C(=O)- oder -S(=O)-. Beispiele für -(C₃-C₁₂)-Heterocycloalkylgruppen Tetrahydrothiophenyl, Tetrhydrofuryl, Tetrahydropyranyl und Dioxanyl.

Der Ausdruck -(C₆-C₂₀)-Aryl und -(C₆-C₂₀)-Aryl-(C₆-C₂₀)-Aryl- umfasst im Sinne der vorliegenden Erfindung mono- oder polycyclische aromatische Kohlenwasserstoffreste. Diese weisen 6 bis 20 Ringatome, besonders bevorzugt 6 bis 14 Ringatome, insbesondere 6 bis 10 Ringatome, auf. Aryl steht vorzugsweise für -(C₆-C₁₀)-Aryl und -(C₆-C₁₀)-Aryl-(C₆-C₁₀)-Aryl-. Aryl steht insbesondere für Phenyl, Naphthyl, Indenyl, Fluorenyl, Anthracenyl, Phenanthrenyl, Naphthacenyl, Chrysenyl, Pyrenyl, Coronenyl. Insbesondere steht Aryl für Phenyl, Naphthyl und Antracenyl.

Substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere (z.B. 1, 2, 3, 4 oder 5) Substituenten aufweisen. Diese Substituenten sind vorzugsweise unabhängig voneinander ausgewählt unter-H, -(C₁-C₁₂)-Alkyl, -O-(C₁-C₁₂)-Alkyl, -O-(C₆-C₂₀)-Aryl, -(C₆-C₂₀)-Aryl, -Halogen (wie Cl, F, Br, I),-COO-(C₁-C₁₂)-Alkyl, -CONH-(C₁-C₁₂)-Alkyl, -(C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; -SO₃Na, -NO₂, -CN, -NH₂, -N[(C₁-C₁₂)-Alkyl]₂. Substituierte -(C₆-C₂₀)-Arylgruppen und -(C₆-C₂₀)-Aryl-(C₆-C₂₀)-Arylgruppen sind vorzugsweise substituierte -(C₆-C₁₀)-Arylgruppen und -(C₆-C₁₀)-Aryl-(C₆-C₁₀)-Arylgruppen, insbesondere substituiertes Phenyl oder substituiertes Naphthyl oder substituiertes Anthracenyl. Substituierte -(C₆-C₂₀)-Arylgruppen tragen vorzugsweise eine oder mehrere z.B. 1, 2, 3, 4 oder 5 Substituenten, ausgewählt unter -(C₁-C₁₂)-Alkylgruppen, -(C₁-C₁₂)-Alkoxygruppen.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹, R², R³, R⁴, R⁵, R⁶, R⁷, R⁸ ausgewählt aus:
-H, -(C₁-C₁₂)-Alkyl, -O-(C₁-C₁₂)-Alkyl, -O-(C₆-C₂₀)-Aryl.

In einer Ausführungsform stehen R⁴ und R⁵ für -H.

In einer Ausführungsform sind R¹', R²', R³', R⁴', R⁵', R⁶', R⁷', R⁸', R¹", R²", R³" , R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₀)-Aryl, -S-Alkyl, -S-Aryl.

In einer Ausführungsform sind R¹', R²', R³', R⁴', R⁵', R⁶', R⁷', R⁸', R¹", R²" , R³", R⁴", R⁵", R⁶", R⁷", R⁸" ausgewählt aus:
-H, -(C₁-C₁₂)-Alkyl, -O-(C₁-C₁₂)-Alkyl, -O-(C₆-C₂₆)-Aryl.

In einer Ausführungsform stehen R¹', R²', R³', R⁴', R⁵', R⁶', R⁷', R⁸', R¹", R²", R³", R⁴", R⁵", R⁶", R⁷", R⁸" für -H.

In einer Ausführungsform stehen R⁹', R¹⁰', R¹¹', R¹²', R⁹", R¹⁰", R¹¹", R¹²" für -(C₆-C₂₀)-Aryl.

In einer Ausführungsform stehen R⁹', R¹⁰', R¹¹', R¹²', R⁹", R¹⁰", R¹¹", R¹²" für Phenyl.

In einer Ausführungsform stehen R⁹', R¹⁰', R¹¹', R¹²', R⁹", R¹⁰", R¹¹", R¹²" für den gleichen Rest.

In einer Ausführungsform weist die Verbindung die allgemeine Struktur (**I**) auf.

In einer Ausführungsform weist die Verbindung die allgemeine Struktur (**II**) auf.

Neben den Verbindungen wird auch ein Komplex beansprucht, welcher diese Verbindungen umfasst.

Komplex umfassend:
- eine zuvor beschriebene Verbindung,
- ein Metallatom ausgewählt aus: Rh, Ru, Co, Ir.

In einer bevorzugten Ausführungsform ist das Metall Rh.

Siehe hierzu R. Franke, D. Selent, A. Börner, "Applied Hydroformylation", Chem. Rev., 2012, DOI:10.1021/cr3001803; S. 5688 Schema 12 "General Method for the Preparation of a P-Modified Rh precatalyst" und darin zitierte Literaturstellen sowie P. W. N. M. van Leeuwen, in Rhodium Catalyzed Hydroformylation, P. W. N. M. van Leeuwen, C. Claver (Hrsg.), Kluwer, Dordrecht, 2000 unter anderem S. 48 ff, S.233 ff. und darin zitierte Literaturstellen sowie K.D. Wiese und D. Obst in Top. Organomet. Chem. 2006, 18, 1-13; Springer Verlag Berlin Heidelberg 2006 S. 6 ff sowie darin zitierte Literaturstellen.

Des Weiteren wird die Verwendung der Verbindung als Ligand in einem Ligand-Metall-Komplex zur Katalyse einer Hydroformylierungsreaktion beansprucht.

Verwendung einer zuvor beschriebenen Verbindung in einem Ligand-Metall-Komplex zur Katalyse einer Hydroformylierungsreaktion.

Das Verfahren bei dem die Verbindung als Ligand in einem Ligand-Metall-Komplex zur Umsetzung eines Olefins zu einem Aldehyd eingesetzt wird, wird ebenfalls beansprucht.

Verfahren umfassend die Verfahrensschritte:
a) Vorlegen eines Olefins,
b) Zugabe eines zuvor beschriebenen Komplexes,
   oder einer zuvor beschriebenen Verbindung und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

Hierbei können die Verfahrensschritte a) bis d) in beliebiger Reihenfolge erfolgen.

Es kann hierbei auch ein Überschuss an Liganden verwendet werden und nicht zwangläufig jeder Ligand liegt gebunden in Form eines Ligand-Metall-Komplexes vor, sondern ist als freier Ligand im Reaktionsgemisch enthalten.

Die Reaktion wird bei üblichen Bedingungen durchgeführt.

Bevorzugt sind eine Temperatur von 80 °C bis 200 °C und ein Druck von 1 bar bis 300 bar. Besonders bevorzugt sind eine Temperatur von 100 °C bis 160 °C und ein Druck von 15 bar bis 250 bar.

Die Edukte für die Hydroformylierung gemäß dem Verfahren der Erfindung sind Olefine oder Gemische von Olefinen, insbesondere Monoolefine mit 2 bis 24, bevorzugt 3 bis 16, besonders bevorzugt 3 bis 12 Kohlenstoffatomen mit end- oder innenständigen C-C-Doppelbindungen, wie z.B. 1-Propen, 1- oder 2-Penten, 2-Methyl-1-buten, 2-Methyl-2-buten, 3-Methyl-1-buten, 1-, 2- oder 3,-Hexen, das bei der Dimerisierung von Propen anfallende C₆-Olefingemisch (Dipropen), Heptene, 2- oder 3-Methyl-1-hexene, Octene, 2-Methylheptene, 3-Methylheptene, 5-Methyl-2-hepten, 6-Methyl-2-hepten, 2-Ethyl-1-hexen, das bei der Dimerisierung von Butenen anfallende C₈-Olefingemisch (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung oder der Trimerisierung von Butenen anfallende C₁₂-Olefingemisch (Tetrapropen oder Tributen), Tetradecene, Hexadecene, das bei der Tetramerisierung von Butenen anfallende C₁₆-Olefingemisch (Tetrabutan) sowie durch Cooligomerisierung von Olefinen mit unterschiedlicher Anzahl von Kohlenstoffatomen (bevorzugt 2 bis 4) hergestellte Olefingemische.

Weiterhin ist es möglich, Gemische aus mehrfach ungesättigten Kohlenwasserstoffen zu hydroformylieren.
Die ungesättigten Verbindungen, welche in dem erfindungsgemäßen Verfahren hydroformyliert werden, umfassen weiterhin ungesättigte Carbonsäurederivate. In besonderen Ausführungsformen sind diese ungesättigten Carbonsäurederivate ausgewählt unter Fettsäureestern.

Im Folgenden wird die Erfindung anhand von Ausführungsbeispielen und einer Figur näher erläutert.

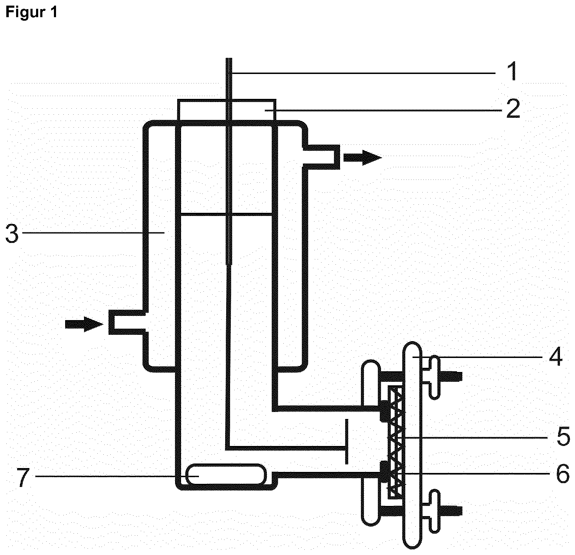

Die Figur 1 zeigt eine Reaktionsapparatur, in welcher die Kupplungsreaktion zu den entsprechenden unsymmetrischen Biarylen durchgeführt werden kann. Die Apparatur umfasst eine Nickelkathode (1) und eine Anode aus Bor-dotiertem Diamant (BDD) auf Silizium (5). Die Apparatur kann mit Hilfe des Kühlmantels (3) gekühlt werden. Die Pfeile deuten hierbei die Durchflussrichtung des Kühlwassers an. Der Reaktionsraum ist mit einem Teflonstopfen (2) verschlossen. Das Reaktionsgemisch wird durch ein Magnetrührstäbchen (7) durchmischt. Auf der anodischen Seite wird die Apparatur durch Schraubzwingen (4) und Dichtungen (6) verschlossen.

### Analytik

### Chromatographie

Die präparativen flüssigkeitschromatographischen Trennungen via "Flashchromatographie" wurden mit einem Maximaldruck von 1.6 bar an Kieselgel 60 M (0.040-0.063 mm) der Firma Macherey-Nagel GmbH & Co, Düren durchgeführt. Die Trennungen ohne Druckbeaufschlagung wurden an Kieselgel Geduran Si 60 (0.063-0.200 mm) der Firma Merck KGaA, Darmstadt durchgeführt. Die als Eluentien verwendeten Lösungsmittel (Essigsäureethylester (technisch), Cyclohexan (technisch)) wurden zuvor destillativ am Rotationsverdampfer gereinigt.

Zur Dünnschichtchromatographie (DC) wurden PSC-Fertigplatten Kieselgel 60 F254 der Firma Merck KGaA, Darmstadt verwendet. Die Rf-Werte sind in Abhängigkeit vom verwendeten Laufmittelgemisch angegeben. Zur Anfärbung der DC-Platten wurde eine Cer-Molybdatophosphorsäure-Lösung als Tauchreagenz verwendet. Cer-Molybdatophosphorsäure-Reagenz: 5.6 g Molybdatophosphorsäure, 2.2 g Cer(IV)-sulfat-Tetrahydrat und 13.3g konzentrierte Schwefelsäure auf 200 mL Wasser.

### Gaschromatographie (GC/GCMS)

Die gaschromatographischen Untersuchungen (GC) von Produktgemischen und Reinsubstanzen erfolgte mit Hilfe des Gaschromatographen GC-2010 der Firma Shimadzu, Japan. Es wird an einer Quarzkapillarsäule HP-5 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min) gemessen. Gaschromatographische Massenspektren (GCMS) von Produktgemischen und Reinsubstanzen wurden mit Hilfe des Gaschromatographen GC-2010 kombiniert mit dem Massendetektor GCMS-QP2010 der Firma Shimadzu, Japan aufgenommen. Es wird an einer Quarzkapillarsäule HP-1 der Firma Agilent Technologies, USA (Länge: 30 m; Innendurchmesser: 0.25 mm; Filmdicke der kovalent gebundenen stationären Phase: 0.25 µm; Trägergas: Wasserstoff; Injektortemperatur: 250 °C; Detektortemperatur: 310 °C; Programm: Methode "hart": 50 °C Starttemperatur für 1 min, Heizrate: 15 °C/min, 290 °C Endtemperatur für 8 min; GCMS: Temperatur der lonenquelle: 200 °C) gemessen.

### Schmelzpunkte

Schmelzpunkte wurden mit Hilfe des Schmelzpunktbestimmungsgerätes SG 2000 der Firma HW5, Mainz gemessen und sind unkorrigiert.

### Elementaranalyse

Die Elementaranalysen wurden in der analytischen Abteilung des Institutes für Organische Chemie der Johannes Gutenberg-Universität Mainz an einem Vario EL Cube der Firma Foss-Heraeus, Haunau angefertigt.

### Massenspektrometrie

Alle Elektrosprayionisation-Messungen (ESI+) wurden an einem QTof Ultima 3 der Firma Waters Micromasses, Milford, Massachusetts durchgeführt. El-Massenspektren sowie die hochaufgelösten El-Spektren wurden an einem Gerät des Typs MAT 95 XL Sektorfeldgerät der Firma ThermoFinnigan, Bremen, gemessen.

### NMR-Spektroskopie

Die NMR-spektroskopischen Untersuchungen wurden an Multikernresonanzspektrometern des Typs AC 300 oder AV II 400 der Firma Bruker, Analytische Messtechnik, Karlsruhe, durchgeführt. Als Lösungsmittel wurde CDCl3 verwendet. Die 1H- und 13C-Spektren wurden gemäß dem Restgehalt an nicht deuteriertem Lösungsmittel nach der NMR Solvent Data Chart der Fa. Cambridge Isotopes Laboratories, USA, kalibriert. Die Zuordnung der 1H- und 13C-Signale erfolgte teilweise mit Hilfe von H,H-COSY, H,H-NOESY, H,C-HSQC und H,C-HMBC-Spektren. Die chemischen Verschiebungen sind als δ-Werte in ppm angegeben. Für die Multiplizitäten der NMR-Signale wurden folgende Abkürzungen verwendet: s (Singulett), bs (breites Singulett), d (Dublett), t (Triplett), q (Quartett), m (Multiplett), dd (Dublett von Dublett), dt (Dublett von Triplett), tq (Triplett von Quartett). Alle Kopplungskonstanten J wurden mit der Anzahl der eingeschlossenen Bindungen in Hertz (Hz) angegeben. Die bei der Signalzuordnung angegebene Nummerierung entspricht der in den Formelschemata angegebenen Bezifferung, die nicht mit der IUPAC-Nomenklatur übereinstimmen muss.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

### Synthese der Chlorophosphite

6-Chlorodibenzo[*d*,*f*][1,3,2]dioxaphosphepin wurde nach DE 10 2008 043 584 und 2-Chloro-4,4,5,5-tetraphenyl-1,3,2-dioxaphospholan nach DE 10 2006 058 682 hergestellt.

### Synthese der unsymmetrischen Biaryle

Die unsymmetrischen Biaryle wurden durch ein elektrochemisches Verfahren durch Kupplung von zwei Phenolen bzw. von einem Naphthol mit einem Phenol, welche sich in ihrem Oxidationspotential unterscheiden, hergestellt. Siehe hierzu auch B. Elsler, D. Schollmeyer, K. M. Dyballa, R. Franke, S. R. Waldvogel, "Metall- und reagensfreie hochselektive anodische Kreuzkupplung von Phenolen", Angew. Chem., 2014, DOI: 10.1002/ange.201400627

### Allgemeine Arbeitsvorschrift:

Die Kupplungsreaktion wurde in einer Apparatur durchgeführt, wie sie in Figur 1 dargestellt ist. 5 mmol des ersten Phenols mit einem Oxidationspotential *E_{Ox}1* werden mit 15 mmol des zweiten Phenols mit einem Oxidationspotential *E_{Ox}2* in den in der nachfolgenden Tabelle 1 angegebenen Mengen in 1,1,1,3,3,3-Hexafluorisopropanol (HFIP) und MeOH oder in Ameisensäure und MeOH gelöst. Die Elektrolyse erfolgt galvanostatisch. Der Außenmantel der Elektrolysezelle wird über einen Thermostaten auf etwa 10 °C temperiert, während die Reaktionsmischung gerührt und auf 50 °C mit Hilfe eines Sandbades erhitzt wird. Nach Ende der Elektrolyse wird der Zellinhalt mit Toluol in einen 50 mL Rundhalskolben überführt und das Lösungsmittel unter vermindertem Druck am Rotationsverdampfer bei 50 °C, 200-70 mbar entfernt. Nicht umgesetztes Edukt wird mittels Kurzwegdestillation zurückerhalten (100 °C, 10⁻³ mbar).

### Elektrodenmaterial

| | |
|---|---|
| Anode: | Bor-dotierter Diamant (BDD) auf Si |
| Kathode: | Ni-Netz |

### Elektrolysebedingungen:

| | |
|---|---|
| Temperatur [T]: | 50 °C |
| Stromstärke [I]: | 15 mA |
| Stromdichte [j]: | 2.8 mA/cm² |
| Ladungsmenge [Q]: | 2 F/mol Unterschusskomponente |
| Klemmspannung [Uₘₐₓ]: | 3-5 V |

Die Synthese der Biaryle erfolgte gemäß der oben beschriebenen allgemeinen Arbeitsvorschrift, und in einer Reaktionsapparatur, wie sie in Figur 1 dargestellt ist.

Die erfindungsgemäßen Verbindungen weisen eine 2,3'-Biphenol-Einheit als Zentral-Baustein auf.

### 2,5'-Dihydroxy-4',5-dimethoxy-2'-methylbiphenyl

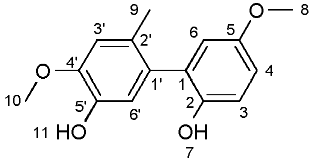

Es wurden 1.66 g (12 mmol, 1.0 Äquiv.) 4-Methylguajacol und 4.49 g (36 mmol, 3.0 Äquiv.) 4-Methoxyphenol in 80 mL HFIP gelöst, 1.63 g MTES zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 2.05 g (66%, 7.9 mmol)
GC (Methode *hart,* HP-5): t_{R}= 14.03 min
R_{f}(CH:EE= 4:1)= 0.33
mₚ= 118.7 °C (aus DCM/CH umkristallisiert)
¹H-NMR (600 MHz, DMSO) δ= 2.01 (s, 3H, 9-H), 3.66 (s, 3H, 8-H), 3.77 (s, 3H, 10-H), 6.53 (d, 1H, 6-H), 6.55 (s, 1H, 6'-H), 6.72 (dd, 1H, 4-H), 6.77 (s, 1H, 3'-H), 6.79 (d, 1H, 3-H), 8.73 (s, 1H, 11-H), 8.75 (s, 1H, 7-H);
Kopplungen: ³J_{3-H, 4-H}= 8.7 Hz; ⁴J_{4-H, 6-H}= 3.0 Hz
¹³C-NMR (151 MHz, DMSO) δ= 19.33 (C-9), 55.32 (C-8), 55.73 (C-10), 113.24 (C-4), 113.75 (C-3'), 115.99 (C-3), 116.07 (C-6), 117.40 (C-6'), 126.56 (C-2'), 129.06 (C-1), 130.95 (C-1'), 143.80 (C-5'), 146.52 (C-4'), 148.29 (C-2), 151.81 (C-5).
HRMS für C₁₅H₁₆O₄ (ESI+) [M+Na⁺]: ber: 283.0946, gef.: 283.0942
MS (El, GCMS): m/z(%): 260 (100) [M]⁺˙, 245 (12) [M-CH₃˙]⁺.
Elementaranalyse für C₁₅H₁₆O_{4:} ber: 69.22%, H: 6.20%, gef.: C: 69.02%, H: 6.34%.

### 3,4'-di-tert-butyl-5,6'-dimethoxy-[1,1'-biphenyl]-2,3'-diol

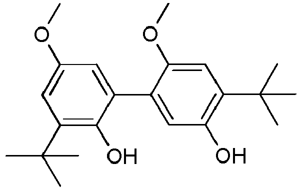

Die Synthese erfolgte nach der obigen allgemeinen Arbeitsvorschrift. Das Produkt konnte nach säulechromatografischer Aufreinung in 15%-iger Ausbeute erhalten werden.
Für einen alternativen Herstellweg siehe: Anwar A. Hamama, Wassef W. Nawar Journal of Agricultural and Food Chemistry 1991, 36, 1063-1069.

Die erfindungsgemäßen Verbindungen können 2,2'-Biphenol-Einheiten als Flügel-Bausteine aufweisen.

### 2,2'-Dihydroxy-4',5-dimethyl-5'-(methylethyl)-3-methoxybiphenyl

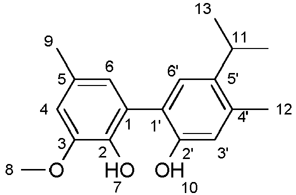

Es wurden 0.69 g (5 mmol, 1.0 Äquiv.) 4-Methylguajacol und 2.28 g (15 mmol, 3.0 Äquiv.) 3-Methyl-4-(methylethyl)phenol in 33 mL 1,1,1,3,3,3-Hexafluorisopropanol (HFIP) gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 716 mg (50%, 2.5 mmol)
GC (Methode *hart,* HP-5): t_{R}= 14.87 min
R_{f}(CH:EE= 4:1)= 0.43
mₚ= 126.8°C (aus CH umkristallisiert)
¹H-NMR (600 MHz, DMSO) δ= 1.17-1.12 (m, 6H, 13-H), 2.24 (m, 6H, 9-H/12-H), 3.01 (dt, 1H, 11-H), 3.79 (s, 3H, 8-H), 6.55 (s, 1H, 6-H), 6.66 (d, 1H, 6'-H), 6.73 (d, 1H, 4-H), 6.96 (s, 1H, 3'-H), 8.16 (s, 1H, 7-H), 8.84 (s, 1H, 10-H);
Kopplungen: ⁴J_{4-H, 6-H}= 2.2 Hz, ⁴J_{6'-H, 11-H}= 2.9 Hz, ³J_{11-H, 13-H}= 6.8 Hz;
¹³C-NMR (151 MHz, DMSO) δ= 18.73, 20.80 (C-9/C-12), 23.54 (C-13), 28.10 (C-11), 55.78 (C-8), 111.23 (C-4), 117.34 (C-6'), 123.42 (C-1'), 123.49 (C-6), 126.43 (C-1), 127.36 (C-5), 127.49 (C-3'), 134.40 (C-5'), 136.62 (C-4'), 141.12 (C-2), 147.65 (C-3), 151.69 (C-2').
HRMS für C₁₈H₂₂O₃ (ESI+) [M+Na⁺]: ber: 309.1467, gef.: 309.1457
MS (El, GCMS): m/z(%): 286 (50) [M]⁺, 271 (100) [M-CH₃˙]⁺, 244 (22) [M-C₃H₆˙]⁺.
Elementaranalyse für C₁₈H₂₂O_{3:} ber: C: 75.50%, H: 7.74%, gef.: C: 75.01%, H: 7.70%.

### 2,2'-Dihydroxy-5,5'-dimethyl-3-methoxybiphenyl

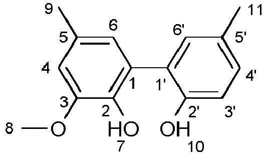

Es wurden 1.66 g (12 mmol, 1.0 Äquiv.) 4-Methylguajacol und 3.91 g (36 mmol, 3.0 Äquiv.) 4-Methylphenol in 65 mL HFIP und 14 mL MeOH gelöst, 1.63 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 440 mg (36%, 1.8 mmol)
GC (Methode *hart,* HP-5): t_{R}= 13.56 min
R_{f}(CH:EE= 4:1)= 0.38
mₚ= 162.0 °C (aus CH umkristallisiert)
¹H-NMR (400 MHz, DMSO) δ= 2.18 (s, 3H, 9-H/11-H), 2.21 (s, 3H, 9-H/11-H), 3.76 (s, 3H, 8-H), 6.53 (s, 1H, 6-H), 6.71 (s, 1H, 4-H), 6.75 (d, 1H, 3'-H), 6.86-6.94 (m, 2H, 4'-H/6'-H), 8.53 (bs, 1H, 7-H/12-H);
Kopplungen: ³J_{3'-H, 4'-H}= 8.4 Hz;
¹³C-NMR (101 MHz, DMSO) δ= 20.21, 20.77 (C-9/C-11), 55.79 (C-8), 111.36 (C-4), 115.69 (C-3'), 123.50 (C-6), 125.72 (C-1'), 126.16 (C-1), 127.20 (C-5), 127.30 (C-5'), 128.50 (C-6'), 131.83 (C-4'), 141.20 (C-2), 147.61 (C-3), 152.11 (C-2').
HRMS für C₁₅H₁₆O₃ (ESI+) [M+Na⁺]: ber: 267.0997, gef.: 267.0999
MS (El, GCMS): m/z(%): 244 (100) [M]⁺˙, 229 (64) [M-CH₃˙]⁺.
Elementaranalyse für C₁₅H₁₆O_{3:} ber: C: 73.75%, H: 6.60%, gef.: C: 73.81%, H: 6.54%.

### 2,2'-Dihydroxy-3-methoxy-3',5,5'-trimethyl-biphenyl

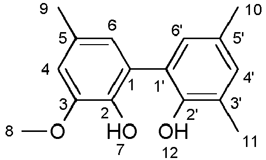

Es wurden 0.70 g (6 mmol, 1.0 Äquiv.) 4-Methylguajacol und 2.08 g (17 mmol, 3.0 Äquiv.) 2,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als leichtgelber Feststoff erhalten.
Ausbeute: 663 mg (45%, 2.5 mmol)
GC (Methode *hart,* HP-5): t_{R}= 13.97 min
R_{f}(CH:EE= 4:1)= 0.29
mₚ= 119.7 °C (aus DCM/CH umkristallisiert)
¹H-NMR (400 MHz, CDCl₃) δ= 2.34 (s, 3H, 10-H), 2.35 (s, 3H, 11-H), 2.38 (s, 3H, 9-H), 3.94 (s, 3H, 8-H), 6.16 (s, 1H, 12-H), 6.20 (s, 1H, 7-H), 6.76 (d, 1H, 4-H), 6.78 (d, 1H, 6-H), 6.98 (d, 1H, 6'-H), 7.03 (d, 1H, 4'-H);
Kopplungen: ⁴J_{4-H, 6-H}= 1.7 Hz, ⁴J_{4'-H, 6'-H}= 2.1 Hz;
¹³C-NMR (101 MHz, CDCl₃) δ= 16.51 (C-9), 20.54 (C-10), 21.20 (C-11), 56.12 (C-8), 110.92 (C-4), 123.95 (C-6), 124.13 (C-1), 124.64 (C-1'), 126.18 (C-3'), 128.82 (C-6'), 129.59 (C-5'), 130.40 (C-5), 131.40 (C-4'), 139.46 (C-2), 146.35 (C-3), 149.42 (C-2').
HRMS für C₁₈H₁₆O₃ (ESI+) [M+Na⁺]: ber: 281.1154, gef.: 281.1152
MS (El, GCMS): m/z(%): 242 (100) [M]⁺, 227 (38) [M-CH₃˙]⁺.
Elementaranalyse für C₁₆H₁₈O_{3:} ber: C: 68.31%, H: 6.45%, gef.: C: 68.29%, H: 6.40%.

### 2,2'-Dihydroxy-3-methoxy-5-methyl-4'-(dimethylethyl)biphenyl

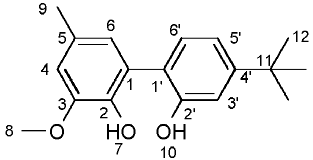

Es wurden 0.69 g (5 mmol, 1.0 Äquiv.) 4-Methylguajacol und 2.25 g (15 mmol, 3.0 Äquiv.) 3-tert-Butylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als farbloser Feststoff erhalten.
Ausbeute: 808 mg (63%, 3.1 mmol)
GC (Methode *hart,* HP-5): t_{R}= 13.97 min
R_{f}(CH:EE= 4:1)= 0.29
mₚ= 160.3 °C (aus DCM/CH umkristallisiert)
¹H-NMR (400 MHz, CDCl₃) δ= 1.37 (s, 9H, 12-H), 2.36 (s, 3H, 9-H), 3.94 (s, 3H, 8-H), 6.25 (s, 1H, 7-H), 6.48 (s, 1H, 10-H), 6.75 (d, 1H, 6-H), 6.79 (d, 1H, 4-H), 7.08 (dd, 1H, 5'-H), 7.12 (d, 1H, 3'-H), 7.27 (d, 1H, 6'-H);
Kopplungen: ⁴J_{4-H, 6-H}= 1.7 Hz; ³J_{5'-H, 6'-H}= 8.0 Hz, ⁴J_{3'-H, 5'-H}= 1.7 Hz;
¹³C-NMR (101 MHz, CDCl₃) δ= 21.24 (C-9), 31.31 (C-12), 34.58 (C-11), 56.15 (C-8), 110.79 (C-4), 114.94 (C-3'), 118.30 (C-5'), 122.37 (C-1'), 123.88 (C-1), 123.94 (C-6), 130.45 (C-6'), 130.53 (C-4'), 139.24 (C-5), 146.32 (C-3), 152.91 (C-2'), 153.13 (C-2).
HRMS für C₁₅H₁₆O₄ (ESI+) [M+Na⁺]: ber: 309.1467, gef.: 309.1466
MS (El, GCMS): m/z(%): 242 (100) [M]⁺, 227 (38) [M-CH₃˙]⁺.
Elementaranalyse für C₁₈H₂₂O_{3:} ber: 75.50%, H: 7.74%, gef.: C: 75.41%, H: 7.72%.

### 2,2'-Dihydroxy-4',5-dimethy-3-methoxylbiphenyl

Es wurden 0.70 g (5 mmol, 1.0 Äquiv.) 4-Methylguajacol und 1.65 g (15 mmol, 3.0 Äquiv.) 3-Methylphenol in 33 mL HFIP gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und zwei Kreuzkupplungsprodukte als farblose Feststoffe erhalten. 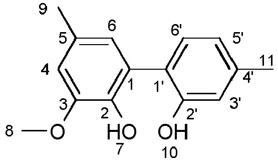 Ausbeute: 266 mg (21%, 1.1 mmol)
GC (Methode *hart,* HP-5): t_{R}= 13.72 min
R_{f}(CH:EE= 4:1)= 0.25
mₚ= 136.2 °C (aus DCM/CH umkristallisiert)
¹H-NMR (400 MHz, CDCl₃) δ= 2.35 (s, 3H, 9-H/11-H), 2.37 (s, 3H, 9-H/11-H), 3.94 (s, 3H, 8-H), 6.17 (s, 1H, 10-H), 6.35 (s, 1H, 2-H), 6.74 (d, 1H, 4-H), 6.76 (s, 1H, 6-H), 6.88-6.83 (m, 1H, 5'-H), 6.90 (d, 1H, 3'-H), 7.21 (d, 1H, 6'-H);
Kopplungen: ⁴J_{4-H, 6-H}= 1.8 Hz, ³J_{5'-H, 6'-H}= 7.7 Hz, ⁴J_{3'-H, 5'-H}= 1.5 Hz;
¹³C-NMR (101 MHz, CDCl₃) δ= 21.11, 21.20 (C-9/C-11), 56.13 (C-8), 110.81 (C-4), 118.25 (C-3'), 121.97 (C-5'), 122.39 (C-1), 123.77 (C-1'), 123.85 (C-6), 130.50 (C-5), 130.68 (C-6'), 139.30 (C-4'), 139.54 (C-2), 146.31 (C-3), 153.33 (C-2').
HRMS für C₁₅H₁₆O₃ (ESI+) [M+Na⁺]: ber: 267.0997, gef.: 267.1006
MS (El, GCMS): m/z(%): 244 (100) [M]⁺, 229 (18) [M-CH₃˙]⁺'.
Elementaranalyse für C₁₅H₁₆O_{3:} ber: C: 73.75%, H: 6.60%, gef.: C: 73.70%, H: 6.68%.

### 2,2'-Dihydroxy-3-methoxy-4'-5,5'-trimethylbiphenyl

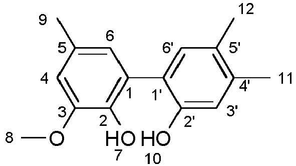

Es wurden 0.69 g (5 mmol, 1.0 Äquiv.) 4-Methylguajacol und 1.83 g (15 mmol, 3.0 Äquiv.) 3,4-Dimethylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 688 mg (52%, 2.6 mmol)
GC (Methode *hart,* HP-5): t_{R}= 14.52 min
R_{f}(CH:EE= 4:1)= 0.29
mₚ= 152.3 °C (aus DCM/CH umkristallisiert)
¹H-NMR (400 MHz, CDCl₃) δ= 12.25 (s, 3H, 11-H), 2.28 (s, 3H, 12-H), 2.36 (s, 3H, 9-H), 3.93 (s, 3H, 8-H), 6.19 (s, 1H, 7-H), 6.25 (s, 1H, 10-H), 6.73 (d, 1H, 4-H), 6.76 (s, 1H, 6-H), 6.88 (s, 1H, 3'-H), 7.08 (s, 1H, 6'-H);
Kopplungen: ⁴J_{4-H, 6-H}= 1.7 Hz;
¹³C-NMR (101 MHz, CDCl₃) Ö= 18.89 (C-11), 19.60 (C-12), 21.24 (C-9), 56.14 (C-8), 110.74 (C-4), 118.93 (C-3'), 122.54 (C-1), 123.82 (C-6), 123.97 (C-1'), 129.03 (C-5), 130.46 (C-4'), 131.69 (C-6'), 137.94 (C-5'), 139.26 (C-2), 146.31 (C-3), 151.36 (C-2').
HRMS für C₁₆H₁₈O₃ (ESI+) [M+Na⁺]: ber: 281.1154, gef.: 281.1157
MS (El, GCMS): m/z(%): 258 (100) [M]⁺, 243 (10) [M-CH₃˙]⁺.
Elementaranalyse für C₁₆H₁₈O_{3:} ber: 74.39%, H: 7.02%, gef.: C: 74.32%, H: 7.20%

### 2,2'-Dihydroxy-5'-isopropyl-3-methoxy-5-methylbiphenyl

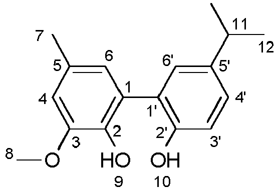

Es wurden 0.69 g (5 mmol, 1.0 Äquiv.) 4-Methylguajacol und 2.05 g (15 mmol, 3.0 Äquiv.) 4-Isopropylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 0.53 g (39%, 1.9 mmol)
GC (Methode *hart,* HP-5): t_{R}= 14.23 min
R_{f}(CH:EE= 4:1)= 0.30
¹H-NMR (400 MHz, CDCl₃) δ= 1.27 (m, 6H), 2.36 (s, 3H), 2.91 (dt, J= 13.8, 6.9, 6.9 Hz, 1H), 3.94 (s, 3H), 6.13-6.27 (m, 2H), 6.82-6.65 (m, 1H), 6.25 (m, 2H), 6.75 (s, 1H), 6.77 (s, 1H), 6.99 (d, J= 8.1 Hz, 1H), 7.19-7.12 (m, 2H);
¹³C-NMR (101 MHz, CDCl₃) δ= 21.25, 24.27, 33.40, 56.18, 110.92, 117.60, 123.91, 124.23, 125.07, 127.29, 128.80, 130.57, 139.29, 141.42, 146.31, 151.51.
HRMS für C₁₇H₂₀O₃ (ESI+) [M+Na⁺]: ber: 295.1310, gef.: 295.1297
MS (El, GCMS): m/z(%): 272 (80) [M]⁺, 257 (100) [M-CH₃˙]⁺.

### 2,2'-Dihydroxy-3-methoxy-5-methyl-5'-tert-butylbiphenyl

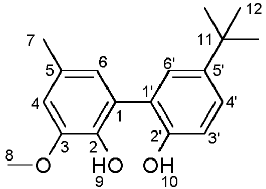

Es wurden 0.69 g (5 mmol, 1.0 Äquiv.) 4-Methylguajacol und 2.26 g (15 mmol, 3.0 Äquiv.) 4-*Tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.48 g (34%, 1.7 mmol)
GC (Methode *hart,* HP-5): t_{R}= 14.52 min
R_{f}(CH:EE= 4:1)= 0.24
¹H-NMR (400 MHz, CDCl₃) δ= 1.34 (s, 9H), 2.37 (s, 3H), 3.94 (s, 3H), 6.17 (s, 1H), 6.24 (s, 1H), 6.75 (s, 1H), 6.77 (s, 1H), 6.99 (d, J= 8.4 Hz, 1H), 7.31-7.29 (m, 1H), 7.33 (dd, J= 8.4, 2.5 Hz, 1H).
¹³C-NMR (101 MHz, CDCl₃) δ= 21.28, 31.61, 34.20, 56.18, 110.91, 117.25, 123.92, 124.41, 124.63, 126.38, 127.78, 130.58, 139.32, 143.70, 146.32, 151.22.
HRMS für C₁₈H₂₂O₃ (ESI+) [M+Na⁺]: ber: 309.1467, gef.: 309.1476
MS (El, GCMS): m/z(%): 286 (28) [M]⁺, 271 (100) [M-CH₃˙]⁺.

### 2,2'-Dihydroxy-3',5'-di-tert-butyl-5-methyl-3-methoxybiphenyl

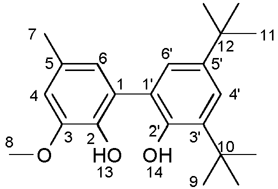

Es wurden 0.69 g (5 mmol, 1.0 Äquiv.) 4-Methylguajacol und 3.12 g (15 mmol, 3.0 Äquiv.) 2,4-Di-*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 9:1 (Cyclohexan:Essigsäureethylester) aufgereinigt und das Produkt als farbloser Feststoff erhalten.
Ausbeute: 0.41 g (24%, 1.2 mmol)
GC (Methode*hart*, HP-5): t_{R}= 15.15 min
R_{f}(CH:EE= 9:1)= 0.35
mₚ= 120.2 °C (inn-Pentan umkristallisiert)
¹H-NMR (400 MHz, CDCl₃) δ= 1.36 (s, 9H), 1.50 (s, 9H), 2.38 (s, 3H), 3.96 (s, 3H), 6.00 (s, 1H), 6.05 (s, 1H), 6.77 (s, 1H), 7.16 (d, J= 2.5 Hz, 1H), 7.39 (d, J= 2.5 Hz, 1H).
¹³C-NMR (101 MHz, CDCl₃) δ= 21.23, 29.88, 31.69, 34.40, 35.23, 56.17, 111.03, 123.96, 124.17, 125.09, 125.50, 130.42, 136.73, 139.72, 142.36, 146.45, 149.82.
MS (El, GCMS): m/z(%): 342 (22) [M]⁺, 327 (100) [M-CH₃˙]⁺.

### 2,2'-Dihydroxy-3',5-dimethyl-3-methoxy-5'-tert-butylbiphenyl

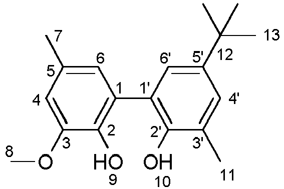

Es wurden 0.69 g (5 mmol, 1.0 Äquiv.) 4-Methylguajacol und 2.47 g (15 mol, 3.0 Äquiv.) 2-Methyl-4-*tert*-butylphenol in 27 mL HFIP und 6 mL MeOH gelöst, 0.68 g Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbliches Öl erhalten.
Ausbeute: 0.69 g (46%, 2.3 mmol)
GC (Methode *hart,* HP-5): t_{R}= 14.79 min
R_{f}(CH:EE= 4:1)= 0.33
¹H-NMR (400 MHz, CDCl₃) δ= 1.37 (s, 9H), 2.39 (d, J= 2.4 Hz, 6H), 3.94 (s, 3H), 6.15 (s, 1H), 6.17 (s, 1H), 6.77 (s, 1H), 6.79 (s, 1H), 7.17 (d, J= 2.5 Hz, 1H), 7.24 (d, J= 2.4 Hz, 1H); ¹³C-NMR (101 MHz, CDCl₃) δ= 16.90, 21.28, 31.67, 34.12, 56.16, 110.94, 124.02, 124.17, 124.59, 125.41, 125.65, 127.86, 130.47, 139.50, 143.07, 146.40, 149.41.
MS (El, GCMS): m/z(%): 300 (18) [M]⁺˙, 285 (100) [M-CH₃˙]⁺.

### 2,2'-Dihydroxy-3-methoxy-5-methyl-5'-(1-methylethyl)biphenyl

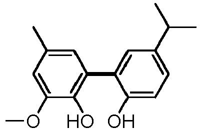

Es wurden 0.69 g (5 mmol, 1.0 eq.) 4-Methylguajacol und 2.05 g (15 mmol, 3.0 eq.) 4-isopropylphenol und 0.68 g Methyltriethylammoniummethylsulfat (MTES) in 27 mL HFIP + 6 mL MeOH Methyltriethylammoniummethylsulfat (MTES) zugegeben und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten.
Ausbeute: 39%, 527 mg, 1.9 mmol.
R_{f} (Cyclohexane:Ethylacetat = 4:1)= 0.30; ¹H NMR (400 MHz, CDCl₃) δ= 1.27 (m, 6H), 2.36 (s, 3H), 2.91 (sept, *J*= 6.9 Hz, 1H), 3.94 (s, 3H), 6.13-6.27 (m, 2H), 6.65-6.82 (m, 2H), 6.99 (d, *J*= 8.1 Hz, 1H), 7.12-7.19 (m, 2H); ¹³C NMR (101 MHz, CDCl₃) δ= 21.37, 24.39, 33.53, 56.31, 111.04, 117.73, 124.04, 124.36, 125.20, 127.42, 128.93, 130.70, 139.42, 141.55, 146.44, 151.64. HRMS für C₁₇H₂₀O₃ (ESI+) [M+Na⁺]: berechnet: 295.1310, gefunden: 295.1297; MS (El, GCMS): m/z(%): 272 (80) [M]⁺, 257 (100) [M-CH₃˙]⁺.

### 2,2'-Dihydroxy-3-methoxy-5-methyl-4'-(methylethyl)biphenyl

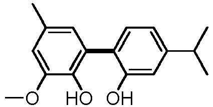

Es wurden 0.69 g (5 mmol, 1.0 eq.) 4-Methylguajacol und 2.065 g (15 mmol, 3.0 eq.) 3-isopropylphenol und 0.68 g Methyltriethylammoniummethylsulfat (MTES) in 33 mL HFIP gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als bräunliches Öl erhalten (Ausbeute: 52%, 705 mg, 2.6 mmol).
R_{f} (Cyclohexane:Ethylacetat = 4:1)= 0.29; ¹H NMR (400 MHz, CDCl₃) δ= 1H NMR (400 MHz, CDCl₃) δ 1.27 (s, 3H), 1.29 (s, 3H), 2.34 (s, 3H), 2.91 (sept, *J*= 7.0 Hz, 1H), 3.94 (s, 3H), 6.15 (s, 1H), 6.35 (s, 1H), 6.73 (d, *J*= 1.8 Hz, 1H), 6.75-6.77 (m, 1H), 6.90 (dd, *J*= 7.9 Hz, 1.8 Hz, 1H), 6.94 (d, *J*= 1.7 Hz, 1H), 7.23 (d, *J*= 7.8 Hz, 1H). ¹³C NMR (101 MHz, CDCl₃) δ= ¹³C NMR (101 MHz, CDCl₃) δ 21.36, 24.02, 33.92, 56.30, 77.16, 110.91, 115.77, 119.56, 122.81, 124.00, 124.08, 130.65, 130.84, 139.38, 146.43, 150.72, 153.54. HRMS für C₁₇H₂₀O₃ (ESI+) [M+Na⁺]: berechnet: 295.1310, gefunden: 295.1305; MS (El, GCMS): m/z(%): 272 (100) [M]⁺˙, 257 (50) [M-CH₃˙]⁺. Elementaranalyse für C₁₇H₂₀O_{3:} berechnet: 74.97%, H: 7.40%, gefunden: C: 75.05%, H: 7.36%.

### 2,2'-Dihydroxy-4',5-dimethyl-3-methoxybiphenyl

Es wurden 0.28 g (2 mmol, 1.0 eq.) 4-Methylguaiacol, 1.22 g (6 mmol, 3.0 eq.) 3-Methylphenyl und 0.77 g MTBS 25 mL HFIP gelöst und der Elektrolyt in die Beaker-typ Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und führte zu den beiden Kreuzkupplungsprodukten als farbloses und viskoses Öl.

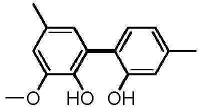

Ausbeute: 21%, 266 mg, 1.1 mmol; R_{f} (Cyclohexane:Ethylacetat = 4:1)= 0.25; mₚ= 136.2 °C (kristallisiert aus Dichloromethan/Cyclohexan); ¹H NMR (400 MHz, CDCl₃) δ= 2.35 (s, 3H), 2.37 (s, 3H,), 3.94 (s, 3H), 6.17 (s, 1H), 6.35 (s, 1H), 6.74 (d, *J*= 1.8 Hz, 1H), 6.76 (s, 1H), 6.88-6.83 (m, 1H), 6.90 (d, 1H, *J*= 1.5 Hz), 7.21 (d, 1H, *J*= 7.7 Hz); ¹³C NMR (101 MHz, CDCl₃) δ= 21.11, 21.20 56.13, 110.81, 118.25, 121.97, 122.39, 123.77, 123.85, 130.50, 130.68, 139.30, 139.54, 146.31, 153.33. HRMS für C₁₅H₁₆O₃ (ESI+) [M+Na⁺]: berechnet: 267.0997, gefunden: 267.1006; MS (El, GCMS): m/z(%): 244 (100) [M]⁺˙, 229 (18) [M-CH₃˙]⁺'. Elementaranalyse für C₁₅H₁₆O_{3:} berechnet: C: 73.75%, H: 6.60%, gefunden: C: 73.70%, H: 6.68%.

### 2,2'-Dihydroxy-5,5'-dimethyl-3'-(1,1-dimethylethyl)-3-methoxybiphenyl

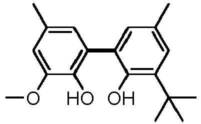

Es wurden 0.69 g (5 mmol, 1.0 eq.) 4-Methylguaiacol, 2.47 g (15 mmol, 3.0 eq.) 4-Methyl-2-*tert*-butylphenol und 0.68 g Methyltriethylammoniummethylsulfat (MTES) in 27 mL HFIP + 6 mL MeOH gelöst gelöst und der Elektrolyt in die Elektrolysezelle überführt. Das Lösungsmittel sowie nicht umgesetzte Eduktmengen werden nach der Elektrolyse unter vermindertem Druck entfernt, das Rohprodukt an Kieselgel 60 als "Flashchromatographie" im Laufmittel 4:1 (Cyclohexan:Essigsäureethylester) aufgereinigt und das Produkt als gelbes Öl erhalten (Ausbeute: 36%, 545 mg, 1.8 mmol).
R_{f} (Cyclohexane:Ethylacetat = 9:1)= 0.36; ¹H NMR (400 MHz, CDCl₃) δ= 1.46 (s, 9H), 2.34 (m, 6H), 3.93 (s, 3H), 5.99 (s, 1H), 6.01 (s, 1H), 6.74 (s, 2H), 6.96 (d, *J*= 1.9 Hz, 1H), 7.14 (d, *J*= 1.9 Hz, 1H); ¹³C NMR (101 MHz, CDCl₃) δ= 21.05, 21.32, 29.96, 35.05, 56.30, 77.16, 111.21, 124.18, 124.24, 125.92, 127.67, 129.15, 129.22, 130.51, 137.57, 139.87, 146.57, 150.10. HRMS für C₂₂H₃₆O₃ (ESI+) [M+Na⁺]: berechnet: 323.1623, gefunden: 323.1618; MS (El, GCMS): m/z(%): 300 (100) [M]⁺˙, 285 (100) [M-CH₃˙]⁺.

### Synthese der Liganden

### 6,6'-((4',5-Dimethoxy-6'-methyl-[1,1'-biphenyl]-2,3'-diyl)bis(oxy))didibenzo[d,f][1,3,2]-dioxaphosphepin.

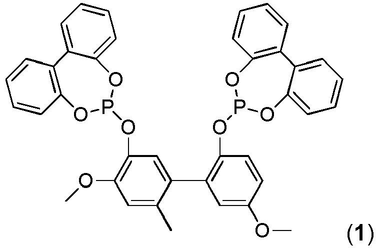

Eine gerührte Suspension von of 4',5-Dimethoxy-6'-methyl-[1,1'-biphenyl]-2,3'-diol (0,602 g; 2,314 mmol) in Toluol (13 ml) wurde mit Triethylamin (0,734 g; 7,254 mmol) versetzt und auf 0°C gekühlt. Zu dieser Mischung wurde eine Lösung von 6-Chlorodibenzo[*d*,*f*][1,3,2]dioxaphosphepin (1,418 g; 5,656 mmol) in Toluol (18 ml) tropfenweise zugegeben. Die Reaktionsmischung wurde für 1 h bei 0 °C und über Nacht bei Raumtemperatur gerührt. Dann wurde die Mischung für 2 h bei 70 °C gerührt und über Kieselgel filtriert. Das Filtrat wurde im Vakuum bis zur Trockne eingeengt und der erhaltene Rückstand durch Säulenchromatografie gereinigt (Dichlormethan/Toluol, 1:1, R*_{f}*= 0,5).
Ausbeute: 0,886 g (1,286 mmol; 56 %).
Elementaranalyse (ber. für C₃₉H₃₀O₈P₂ = 688,57 g/ mol) C 68,16 (68,02); H 4,36 (4,39); P 8,96 (9,00) %.
³¹P-NMR (CD₂Cl₂): 145,7; 146,7 ppm.
¹H-NMR (CD₂Cl₂): 2,21 (m, 3 H); 3,87 (s, 3 H); 4,06 (s, 3 H); 6,87 (m, 1 H, Hₐᵣₒₘ); 6,92-7,14 (m, br, 4 H, Hₐᵣₒₘ); 7,17 (m, 1 H, Hₐᵣₒₘ); 7,28 (m, 1 H, Hₐᵣₒₘ); 7,28-7,38 (m, 7 H, Hₐᵣₒₘ); 7,38-7,42 (m, 2 H, Hₐᵣₒₘ); 7,42-7,48 (m, 1 H, Hₐᵣₒₘ); 7,49-7,61 (m, 4 H, Hₐᵣₒₘ) ppm.
¹³C-NMR (CD₂Cl₂): 20,2; 21,6; 56,1; 56,6; 114,4 (d, *J*_{CP}= 18,7 Hz); 117,0; 122,2 (d, *J*_{CP}= 8,7 Hz); 122,6 (d, *J*_{CP}= 14,2 Hz); 124,2 (d, *J*_{CP}= 6,7 Hz); 125,8 (d, *J*_{CP}= 4,6 Hz); 128,6; 129,4; 129,6 (d, *J*_{CP}= 3,2 Hz); 130,3 (d, *J*_{CP}= 7,3 Hz); 130,4; 131,5 (m); 134,5; 138,4; 138,7 (d, *J*_{CP}= 6,5 Hz); 143,2 (d, *J*_{CP}= 7,6 Hz); 149,4 (m); 150,8; 156,7 ppm.
ESI-TOF/HRMS: m/e 689,14883 (M+H)⁺.

### 2,2'-((4',5-Dimethoxy-6'-methyl-[1,1'-biphenyl]-2,3'-diyl)bis(oxy))bis(4,4,5,5-tetraphenyl-1,3,2-dioxaphospholan)

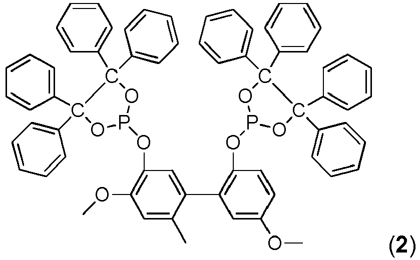

Eine Lösung von 4',5-Dimethoxy-6'-methyl-[1,1'-biphenyl]-2,3'-diol (0,335 g; 1,286 mmol) in THF (10 ml) wurde mit 2 Äquivalenten n-Butyllithium, gelöst in Hexan (4.83 ml), bei -20 °C versetzt. Die Mischung wurde für 20 min bei -20 °C gerührt und anschließend eine Lösung von 2-Chloro-4,4,5,5-tetraphenyl-1,3,2-dioxaphospholan (1,109 g; 2,573 mmol) in THF (6 ml) bei Raumtemperatur zugegeben. Die Reaktionsmischung wurde über Nacht gerührt und das Lösungsmittel im Vakuum abgezogen. Toluol (20 ml) wurde zugegeben und die resultierende Lösung filtriert. Das Filtrat wurde im Vakkum bis zur Trockne eingeengt. Der erhaltene Feststoff wurde für 3 h bei 50 °C / 0,1 mbar getrocknet und dann aus heißem Acetonitril (26 ml) umkristallisiert. Ausbeute: 0,602 g (0,574 mmol; 45 %).
Elementaranalyse (ber. für C₆₇H₅₄O₈P₂ = 1049,10 g/ mol) C 75,66 (76,71); H 5,24 (5,19); P 6,17 (5,90) %.
³¹P-NMR (CD₂Cl₂): 140,6; 141,1 ppm.
¹H-NMR (CD₂Cl₂): 1,99 (m, 3 H); 3,80 (s, 3 H); 3,90 (s, 3 H); 6,50 (m, 1 H, Hₐᵣₒₘ); 6,70 (m, 1 H, Hₐᵣₒₘ); 6,86 (m, 1 H, Hₐᵣₒₘ); 7,02-7,23 (m, 34 H, Hₐᵣₒₘ); 7,41 (m, 4 H, Hₐᵣₒₘ); 7,62 (m, 4 H, Hₐᵣₒₘ) ppm.
¹³C-NMR (CD₂Cl₂, mehrere Regionen im aromatischen Bereich mit Signalüberlappungen): 20,1; 56,0; 56,8; 95,3; 95,5; 113,9; 114,5; 116,5; 122,4 (d, *J*_{CP}= 8,5 Hz); 124,5; 127,4; 127,4; 127,5; 127,7; 129,2; 129,9; 130,1; 130,4; 130,7; 133,9; 134,9; 138,3 (d, *J*_{CP}= 6,7 Hz); 142,5; 142,7; 142,9; 143,1; 143,1; 150,8 (d, *J*_{CP}= 3,2 Hz); 156,3 ppm.
ESI-TOF/HRMS: m/e 1071,31785 (M+Na)⁺.

### 6,6'-((3,4'-Di-tert-butyl-5,6'-dimethoxy-[1,1'-biphenyl]-2,3'-diyl)bis(oxy))-dibenzo[d,f][1,3,2]dioxaphosphepin

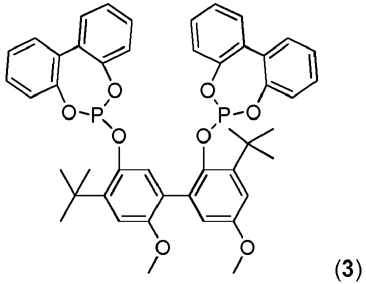

Eine Lösung von 3,4'-Di-*tert*-butyl-5,6'-dimethoxy-[1,1'-biphenyl]-2,3'-diol (0,505 g; 1,409 mmol) in Toluol (10 ml) wurde mit Pyridin (0,251 g; 3,170 mmol) versetzt und die erhaltene Mischung bei 3 °C tropfenweise zu einer Lösung von 6-Chlorodibenzo[*d*,*f*][1,3,2]dioxaphosphepin (0,777 g; 3,100 mmol) in Toluol (10 ml) gegeben. Die Reaktionsmischung wurde über Nacht bei Raumtemperatur und für 2 h bei 70 °C gerührt. Die Mischung wurde über Kieselgel filtriert und das Filtrat im Vakuum bis zur Trockne eingeengt. Der erhaltene Rückstand wurde aus Acetonitril (13 ml) umkristallisiert. Elementaranalyse (ber. für C₄₆H₄₄O₈P₂ = 786,79 g/ mol) C 70,30 (70,22); H 5,78 (5,64); P 7,93 (7,87) %.
³¹P-NMR (CD₂Cl₂): 145,2 (d, *J*_{PP} = 14,3 Hz); 147,4 (d, *J*_{PP} = 14,3 Hz) ppm.
¹H-NMR (CD₂Cl₂): 1,50 (s, 9 H); 1,59 (s, 9 H); 3,81 (m, 3 H); 3,90 (m, 3 H); 6,74 (m, 1 H, Hₐᵣₒₘ); 6,78-6,82 (m, 1 H, Hₐᵣₒₘ); 6,84-6,88 (m, 1 H, Hₐᵣₒₘ); 6,96-7,01 (m, 1 H, Hₐᵣₒₘ); 7,07 (m, 1 H, Hₐᵣₒₘ); 7,11-7,18 (m, 2 H, Hₐᵣₒₘ); 7,24 (m, 1 H, Hₐᵣₒₘ); 7,26-7,35 (m, 7 H, Hₐᵣₒₘ); 7,40 (m, 1 H, Hₐᵣₒₘ); 7,43-7,53 (m, 4 H, Hₐᵣₒₘ) ppm.
¹³C-NMR (CD₂Cl₂): 29,8; 30,9; 35,3; 35,8; 55,9; 56,1; 114,4; 115,0; 116,5; 120,0 (d, *J*_{CP}= 9,6 Hz); 122,4; 122,6; 123,1; 125,5 (d, *J*_{CP}= 4,5 Hz); 125,6; 129,3; 129,4 (d, *J*_{CP}= 4,5 Hz); 129,7 (m); 129,9; 130,0; 130,1; 130,2; 131,3 (d, *J*_{CP}= 2,9Hz); 131,4; 131,5; 131,5; 131,5; 131,6; 131,6 (Die zuletztgenannten sechs Frequenzen resultieren wahrscheinlich auch aus C-P Kopplungen. Eine Zuordnung einzelner Signale ist nicht erfolgt.); 132,1 (d, *J*_{CP}= 6,8 Hz); 140,3; 143,3 (d, *J*_{CP}= 7,9 Hz); 143,7; 143,9 (d, *J*_{CP}= 7,7 Hz); 149,3; 149,3; 149,4; 149,4; 149,5; 149,5; (Die zuletztgenannten sechs Frequenzen resultieren wahrscheinlich auch aus C-P Kopplungen. Eine Zuordnung einzelner Signale ist nicht erfolgt.); 150,0 (d, *J*_{CP}= 6,0 Hz); 155,6 (d, *J*_{CP}= 15,6 Hz) ppm.
ESI-TOF/HRMS: m/e 787.25933 (M+H)⁺.

### 2,2'-((3,4'-Di-tert-butyl-5,6'-dimethoxy-[1,1'-biphenyl]-2,3'-diyl)bis(oxy))bis(4,4,5,5-tetraphenyl-1,3,2-dioxaphospholan)

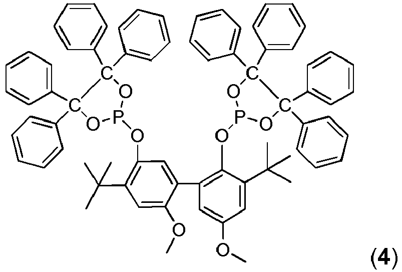

Eine Lösung von 3,4'-Di-*tert*-butyl-5,6'-dimethoxy-[1,1'-biphenyl]-2,3'-diol (0,357 g; 0,996 mmol) in THF (4,5 ml) wurde bei -20 °C mit zwei Äquivalenten n-Butyllithium in Hexan (4 ml) versetzt. Die Mischung wurde noch 20 min bei dieser Temperatur gerührt und dann auf Raumtemperatur erwärmt. Eine Lösung von 2-Chloro-4,4,5,5-tetraphenyl-1,3,2-dioxaphospholan (0,858 g; 1,992 mmol) in THF (7 ml) wurde zugegeben. Die Reaktionsmischung wurde über Nacht gerührt und das Lösungsmittel im Vakuum entfernt. Nach Zugabe von Toluol (20 ml) wurde die resultierende Suspension über Kieselgel filtriert und das Filtrat im Vakuum bis zur Trockne eingeengt. Der erhaltene gelbe Feststoff wurde bei 50 °C / 0,1 mbar getrocknet und dann mittels Säulenchromatografie gereinigt (Hexan/Dichlormethan, 1:1, R*_{f}* = 0,5). Ausbeute: 0,842 g (0,734 mmol; 77 %). Elementaranalyse (ber. für C₇₄H₆₈O₈P₂ = 1047,13 g/ mol) C 78,11 (78,00); H 5,38 (5,39); P 6,02 (5,92) %.
³¹P-NMR (CD₂Cl₂): 140,0 (d, *J*_{PP}= 25,9 Hz); 147,7 (d, *J*_{PP}= 25,9 Hz) ppm.
¹H-NMR (CD₂Cl₂): 1,11 (m, 9 H); 1,33 (m, 9 H); 3,77 (s, 3 H); 3,86 (m, 3 H); 6,57 (m, 1 H, Hₐᵣₒₘ); 6,83 (m, 2 H, Hₐᵣₒₘ); 6,90-7,01 (m, 8 H, Hₐᵣₒₘ); 7,04-7,06 (m, 2 H, Hₐᵣₒₘ); 7,06-7,11 (m, 10 H, Hₐᵣₒₘ); 7,11-7,13 (m, 4 H, Hₐᵣₒₘ); 7,13-7,15 (m, 3 H, Hₐᵣₒₘ); 7,16-7,18 (m, 2 H, Hₐᵣₒₘ); 7,20-7,24 (m, 3 H, Hₐᵣₒₘ); 7,26-7,30 (m, 3 H, Hₐᵣₒₘ); 7,35-7,42 (m, 2 H, Hₐᵣₒₘ); 7,49-7,60 (m, 4 H, Hₐᵣₒₘ) ppm.
¹³C-NMR (CD₂Cl₂): 29,8; 30,5; 35,0; 35,1; 55,7; 55,8; 94,5 (d, *J*_{CP}= 8,3 Hz); 94,7 (d, *J*_{CP}= 7,5 Hz); 94,9 (d, *J*_{CP}= 8,3 Hz); 95,1 (d, *J*_{CP}= 7,7 Hz); 114,0; 116,9 (d, *J*_{CP}= 4,8 Hz); 120,5 (d, *J*_{CP}= 10,2 Hz); überlappende Signale von 127,0 bis 129,5 ppm; 129,8; 130,0; 131,3; 132,7 (d, *J*_{CP}= 9,2 Hz); 139,2; 139,7; 142,2 (d, *J*_{CP}= 5,9 Hz); 142,6; 142,6; 142,7; 142,8; 142,8; 142,9; 142,9; 143,0; 143,4; 143,5; 144,0; 144,0; 144,1; 144,1 (Signalüberlappungen für zuletztgenannte 14 Frequenzen); 155,0 (d, *J*_{CP}= 8,3 Hz); (d, *J*_{CP}= 19,7 Hz) ppm.
ESI-TOF/HRMS: m/e 1169,42807 (M+Na)⁺.

### Arbeitsvorschrift für die Katalyseversuche

Die Hydroformylierung wurde in einem mit Druckkonstanthaltung, Gasflussmessung, Begasungsrührer und Druckpipette ausgestatteten 200 ml-Autoklaven der Fa. Premex Reactor AG, Lengau, Schweiz, durchgeführt. Zur Minimierung eines Einflusses von Feuchtigkeit und Sauerstoff wurde das als Solvens benutzte Toluol mit Natrium-Ketyl getrocknet und unter Argon destilliert. Die als Substrat eingesetzten Substrate 1-Octen (Aldrich), *cis*/*trans*-2-Penten (Aldrich) und n-Octene (Oxeno GmbH, Octenisomerengemisch aus 1-Octen: ∼3 %; *cis*+*trans*-2-Octen; ∼49%; *cis*+*trans*-3-Octen: ∼29 %; *cis*+*trans*-Octen-4: ∼16 %; gerüstisomere Octene: ∼3 %) wurden mehrere Stunden über Natrium am Rückfluß erhitzt und unter Argon destilliert.

Für die Versuche wurden im Autoklaven unter Argonatmosphäre folgende Lösungen des Rhodiums in Form von [(acac)Rh(COD)] (acac=Acetylacetonat-Anion; COD=1,5-Cyclooctadien) (OMG AG & Co. KG, Hanau, DE) als Katalysatorvorstufe in Toluol eingefüllt: Für Versuche mit 100 ppm-m Rhodium 10 ml einer 4,31 millimolaren, für 40 ppm-m die gleiche Menge einer entsprechend verdünnte Lösung. Anschließend wurde die entsprechende Menge der in Toluol gelösten Phosphitverbindung, in der Regel 2 bis 5 Ligandäquivalente pro Rhodium, zugemischt. Durch Zugabe von weiterem Toluol (Die Gesamtmasse an Toluol wurde bestimmt für die GC-Analyse, s.u.) wurde das Anfangsvolumen der Katalysatorlösung auf a) 41,0 ml eingestellt bei beabsichtigter Zugabe von 15 ml des Olefins über die Druckpipette (1-Octen, n-Octenen und Versuche mit erhöhter Konzentration an 2-Penten), oder b) 51,9 ml eingestellt bei beabsichtigter Zugabe von 4,1 ml 2-Penten. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten 9,75 g; 139,00 mmol. Der Autoklav wurde bei einem Gesamtgasdruck (Synthesegas: Linde; H₂ (99,999%) : CO (99,997%) = 1:1) von a) 42 bar für einen Enddruck von 50 bar; b) 12 bar für den Enddruck von 20 bar und c) 7 bar für einen Enddruck von 10 bar unter Rühren (1500 U/min) auf die jeweils angegebenen Temperaturen aufgeheizt. Nach Erreichen der Reaktionstemperatur wurde der Synthesegasdruck auf a) 48,5 bar für einen Enddruck von 50 bar, b) 19,5 bar für einen Enddruck von 20 bar und c) 9,5 bar für einen Enddruck von 10 bar erhöht das jeweils in der Tabelle angegebene Olefin(-gemisch) mit einem in der Druckpipette eingestellten Überdruck von ca. 3 bar zugepresst. Die Reaktion wurde bei konstantem Druck von jeweils 50, 20 bzw. 10 bar (Nachdruckregler der Fa. Bronkhorst, NL) über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 µm, Die quantitative Bestimmung von Restolefin und Aldehyd erfolgte gegen das Lösungsmittel Toluol als internen Standard.

### Ergebnisse der Katalyseversuche

Solvens: Toluol
Ausb. = Ausbeute
Sel. = Selektivität
p = Druck in [bar]
T = Temperatur in [°C]
t = Zeit in [h]
[Rh] = Rhodiumkonzentration in [ppm]
L/Rh = Verhältnis von Ligand zum Rhodium

Als Vergleichsliganden wurden die Liganden **A** und **B** ausgewählt. Ihre Herstellung erfolgte nach DE 10 2006 058 682 A1.

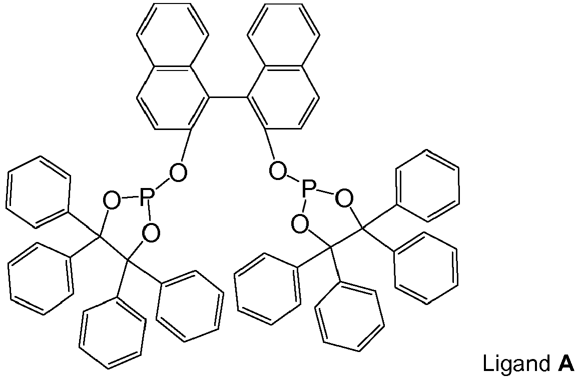

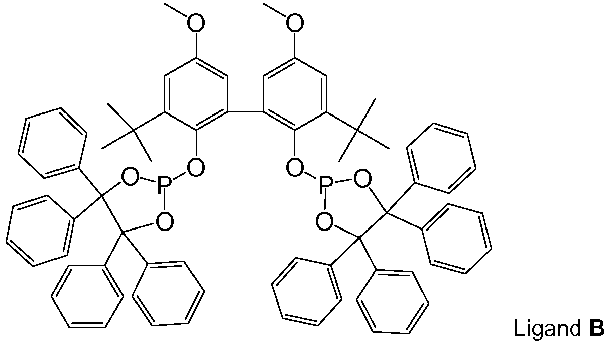

Die erfindungsgemäßen Verbindungen sind hierbei mit * gekennzeichnet.

**Tabelle 1: 1-Octen**

| **Ligand** | **p (bar)** | **T (°C)** | **t (h)** | **[Rh] (ppm)** | **L/Rh** | **Ausb. (%)** | **Sel**. **(%)** |
|---|---|---|---|---|---|---|---|
| **1*** | 50 | 100 | 4 | 40 | 2 | 93 | 90,1 |
| **2*** | 50 | 100 | 4 | 40 | 1 | 99 | 47,1 |
| **3*** | 50 | 100 | 4 | 40 | 2 | 80 | 95,8 |
| **4*** | 50 | 100 | 4 | 40 | 2 | 84 | 96,2 |
| **A** | 50 | 100 | 4 | 40 | 2 | 89 | 83 |

Wie der Tabelle 1 zu entnehmen ist, zeichnen sich die erfindungsgemäßen Verbindungen in der Hydroformylierung von terminalen Olefinen, hier konkreter 1-Octen, durch sehr gute Ausbeuten aus. Die Verbindungen **1** und **2** weißen gegenüber dem Vergleichsliganden **A** eine verbesserte Ausbeute auf. Die Verbindungen **3** und **4,** welche geringfügig schlechtere Ausbeuten wie der Vergleichsligand **A** aufweisen, haben beide aber eine deutlich bessere Selektivität.

Die erfindungsgemäßen Verbindungen geben also die Möglichkeit die Reaktion entweder in der Ausbeute oder in der Selektivität zu verbessern, je nach der entsprechenden Wahl der Verbindung. Durch den Einsatz der Verbindung **1** werden sogar Ausbeute und Selektivität gesteigert.

**Tabelle 2: 2-Penten**

| **Ligand** | **p (bar)** | **T (°C)** | **t (h)** | **[Rh] (ppm)** | **L/Rh** | **Ausb. (%)** | **Sel**. **(%)** |
|---|---|---|---|---|---|---|---|
| **1*** | 20 | 120 | 4 | 100 | 1 | 100 | 54,9 |
| **1*** | 20 | 120 | 4 | 100 | 0,5 | 96 | 57,5 |
| **3*** | 20 | 120 | 4 | 100 | 2 | 93 | 83,3 |
| **B** | 20 | 120 | 4 | 100 | 2 | 14 | 99 |

Mit dem Vergleichsliganden **B** konnte für 2-Penten zwar eine sehr gute Selektivität, 99 %, erzielt werden, jedoch ist die Ausbeute mit 14 % so gering, dass der Einsatz eines solchen Liganden für ein großtechnisches Verfahren nur wenig interessant ist. Die Raum-Zeit-Ausbeuten mit diesem Liganden sind so schlecht, sodass dies aus ökonomischer Sicht gegen den Einsatz des Vergleichsliganden **B** spricht.

Die erfindungsgemäßen Verbindungen weißen alle sehr gute Ausbeute in Kombination mit einer annehmbaren bis guten Selektivität auf.

Weiterhin ist es möglich, bei der Reaktion ein geringes Phosphor - Rhodium-Verhältnis - kurz P / Rh einzustellen. Dies ist von besonderer Relevanz, da die Liganden einen großen Teil der Prozesskosten ausmachen können. Wird also weniger Ligand benötigt, wirkt sich das umgehend positiv auf die Gesamtwirtschaftlichkeit des großtechnischen Prozesses aus.

Wie die Versuchsergebnisse zeigen, wird die gestellte Aufgabe durch die erfindungsgemäßen Verbindungen gelöst.

Es ist erstmalig gelungen, Bisphosphite, die eine 2,3'-Biphenol-Einheit als Zentral-Baustein aufweisen, und die gute bis sehr gute Hydroformylierungseigenschaften aufweisen, bereitzustellen. Dies konnte anhand der gezeigten Beispiele belegt werden.
Solche konkreten Strukturen und derartige Liganden waren bis dato gänzlich unbekannt und nicht zugänglich.
Das besondere hierbei ist, dass ein 2,3'-Biphenol-Zetranbaustein verwendet und somit eine völlig neue Asymmetrie erzeugt wird, die zu unsymmetrischen Bisphosphiten führt.
Diese unsymmetrischen Bisphosphite sind strukturell somit gänzlich von denen im Stand der Technik beschriebenen Bisphoshiten verschieden, bei denen unsymmetrische Bisphosphit-Liganden durch eine bestimmte Anordnung von symmetrischen Biaryl-Bausteinen generiert werden, indem sich beispielsweise die beiden Flügel-Bausteine unterscheiden, die einzelnen Bausteine (Zentral-Baustein und Flügel-Bausteine) an sich aber symmetrisch sind.

## Patentansprüche

1. Verbindung, welche eine der beiden allgemeinen Strukturen **I** oder **II** aufweist: 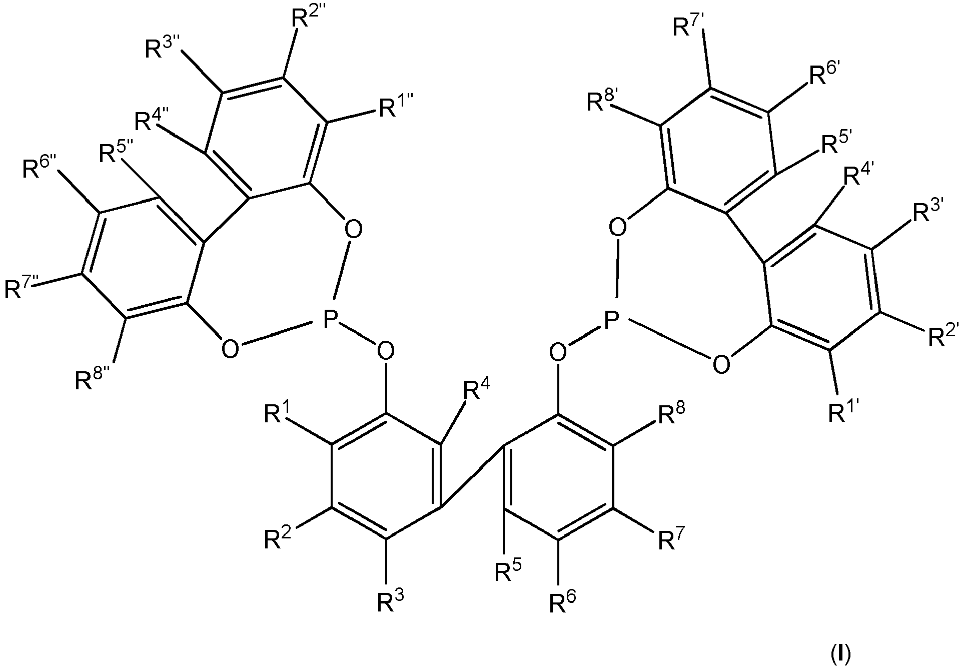 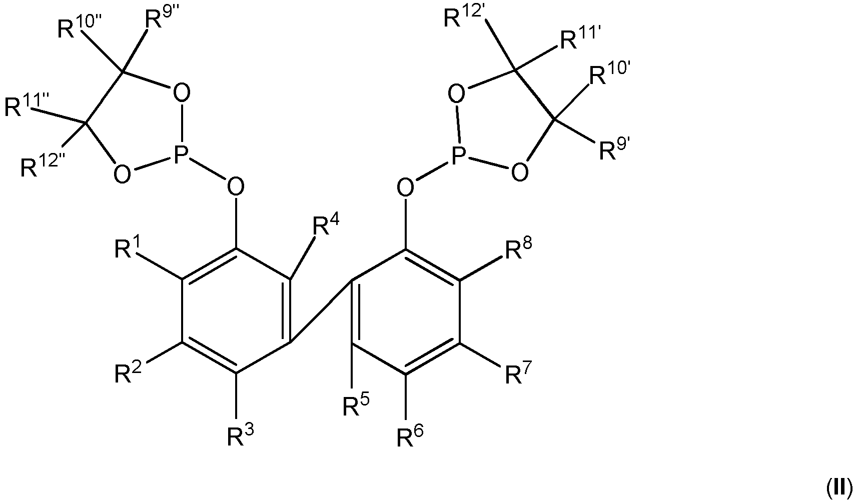 wobei
R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, , -CO-(C₁-C₁₂)-Alkyl, -CO-(C₆-C₂₀)-Aryl, -COOH, -OH, -SO₃H, -CN, -NH₂, -N[(C₁-C₁₂)-Alkyl]₂;
R¹', R²', R³', R⁴', R⁵', R⁶', R⁷', R⁸', R¹", R²", R³", R⁴", R⁵", R⁶", R⁷", R⁸" ausgewählt sind aus:
-H, -(C₁-C₁₂)-Alkyl, -O-(C₁-C₁₂)-Alkyl, -O-(C₆-C₂₀)-Aryl, -(C₆-C₂₀)-Aryl, -S-Alkyl, -S-Aryl, Halogen, COO-(C₁-C₁₂)-Alkyl, CONH-(C₁-C₁₂)-Alkyl, -CO-(C₁-C₁₂)-Alkyl, -CO-(C₆-C₂₀)-Aryl, -COOH, -OH, - SO₃H, -NH₂, -N[(C₁-C₁₂)-Alkyl]₂;
R⁹', R¹⁰', R¹¹', R¹²', R⁹", R¹⁰", R¹¹", R¹²" ausgewählt sind aus:
-H, -(C₆-C₂₀)-Aryl;
wobei die genannten Alkyl- und Arylgruppen wie folgt substituiert sein können:
(C₁-C₁₂)-Alkyl und O-(C₁-C₁₂)-Alkyl können jeweils unsubstituiert oder durch einen oder mehrere gleiche oder verschiedene Reste substituiert sein, die ausgewählt sind unter (C₃-C₁₂)-Cycloalkyl, (C₃-C₁₂)-Heterocycloalkyl, (C₆-C₂₀)-Aryl, Fluor, Chlor, Cyano, Formyl, Acyl oder Alkoxycarbonyl;
substituierte -(C₆-C₂₀)-Arylgruppen und -(C₆-C₂₀)-Aryl-(C₆-C₂₀)-Arylgruppen können, in Abhängigkeit von der Ringgröße, einen oder mehrere Substituenten aufweisen; diese Substituenten sind unabhängig voneinander ausgewählt unter -H, -(C₁-C₁₂)-Alkyl, -O-(C₁-C₁₂)-Alkyl, -O-(C₆-C₂₀)-Aryl, -(C₆-C₂₀)-Aryl, -Halogen, -COO-(C₁-C₁₂)-Alkyl, -CONH-(C₁-C₁₂)-Alkyl, - (C₆-C₂₀)-Aryl-CON[(C₁-C₁₂)-Alkyl]₂, -CO-(C₁-C₁₂)-Alkyl, -CO-(C₆-C₂₀)-Aryl, -COOH, -OH, -SO₃H; - SO₃Na, -NO₂, -CN, -NH₂, -N[(C₁-C₁₂)-Alkyl]₂.

2. Verbindung nach Anspruch 1,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

3. Verbindung nach einem der Ansprüche 1 oder 2,
wobei R¹, R², R³, R⁴, R⁵, R⁶, R⁷, R⁸ ausgewählt sind aus:
-H, -(C₁-C₁₂)-Alkyl, -O-(C₁-C₁₂)-Alkyl, -O-(C₆-C₂₀)-Aryl.

4. Verbindung nach einem der Ansprüche 1 bis 3.
wobei R⁴ und R⁵ für -H stehen.

5. Verbindung nach einem der Ansprüche 1 bis 4,
wobei R⁷', R²', R³', R⁴', R⁵', R⁶', R⁷', R⁸', R¹", R²", R³", R⁴", R⁵", R⁶", R⁷", R⁸" ausgewählt sind aus:
-H, -(C₁-C₁₂)-Alkyl, -O-(C₁-C₁₂)-Alkyl, -O-(C₆-C₂₀)-Aryl, -S-Alkyl, -S-Aryl.

6. Verbindung nach einem der Ansprüche 1 bis 5,
wobei R⁷', R²', R³', R⁴', R⁵', R⁶', R⁷', R⁸', R¹", R²", R³", R⁴", R⁵", R⁶", R⁷", R⁸" ausgewählt sind aus:
-H, -(C₁-C₁₂)-Alkyl, -O-(C₁-C₁₂)-Alkyl, -O-(C₆-C₂₀)-Aryl.

7. Verbindung nach einem der Ansprüche 1 bis 6,
wobei R⁷', R²', R³', R⁴', R⁵', R⁶', R⁷', R⁸', R¹", R²", R³", R⁴", R⁵", R⁶", R⁷", R⁸" für -H stehen.

8. Verbindung nach einem der Ansprüche 1 bis 7,
wobei R⁹', R¹⁰', R¹¹', R¹²', R⁹", R¹⁰", R¹¹", R¹²" für -(C₆-C₂₀)-Aryl stehen.

9. Verbindung nach einem der Ansprüche 1 bis 8,
wobei R⁹', R¹⁰', R¹¹', R¹²', R⁹", R¹⁰", R¹¹", R¹²" für Phenyl stehen.

10. Verbindung nach einem der Ansprüche 1 bis 9,
wobei R⁹', R¹⁰', R¹¹', R¹²', R⁹", R¹⁰", R¹¹", R¹²" für den gleichen Rest stehen.

11. Verbindung nach einem der Ansprüche 1 bis 10,
wobei die Verbindung die allgemeine Struktur (**I**) aufweist.

12. Verbindung nach einem der Ansprüche 1 bis 10,
wobei die Verbindung die allgemeine Struktur (**II**) aufweist.

13. Komplex umfassend:
- eine Verbindung nach einem der Ansprüche 1 bis 12,
- ein Metallatom ausgewählt aus: Rh, Ru, Co, Ir.

14. Verwendung einer Verbindung nach einem der Ansprüche 1 bis 12,
zur Katalyse einer Hydroformylierungsreaktion.

15. Verfahren umfassend die Verfahrensschritte:
a) Vorlegen eines Olefins,
b) Zugabe eines Komplexes nach Anspruch 13,
oder einer Verbindung nach einem der Ansprüche 1 bis 12 und einer Substanz, welche ein Metallatom ausgewählt aus: Rh, Ru, Co, Ir aufweist,
c) Zuführen von H₂ und CO,
d) Erwärmen des Reaktionsgemisches, wobei das Olefin zu einem Aldehyd umgesetzt wird.

## Claims

1. Compound having one of the two general structures **I** and **II:** 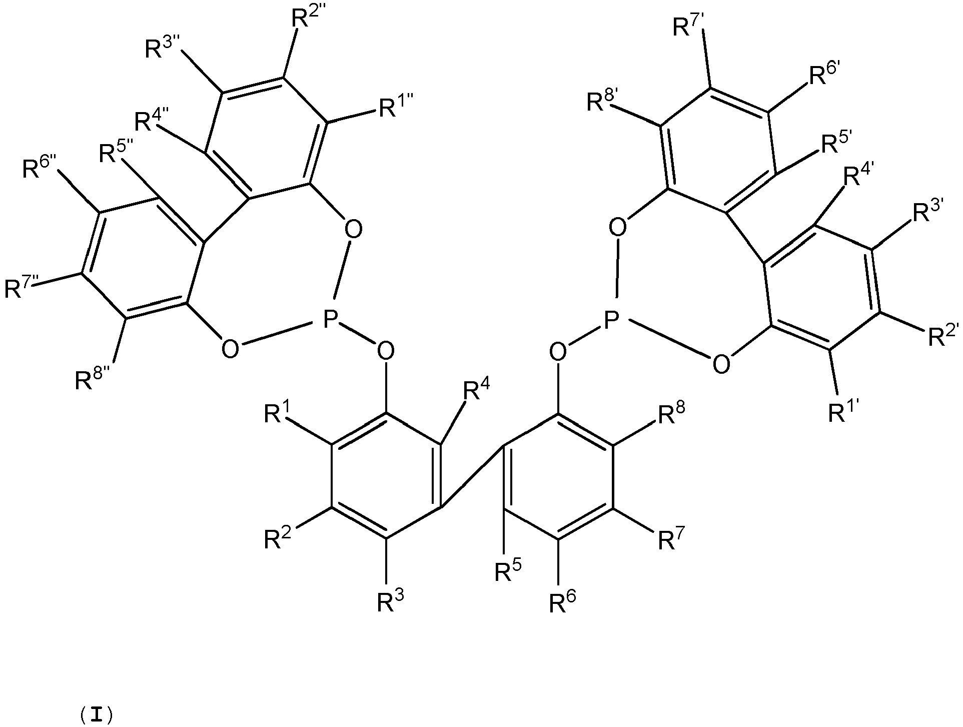 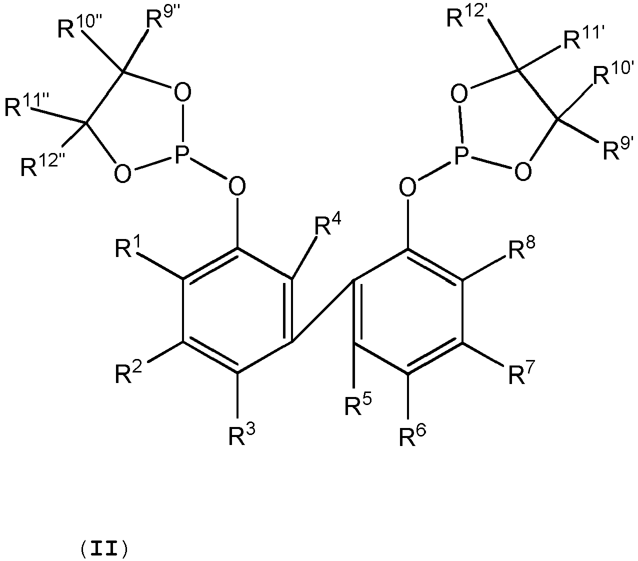 where
R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, - (C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -CN, -NH₂, -N[(C₁-C₁₂)-alkyl]₂;
R¹', R²', R³', R⁴', R⁵', R⁶', R⁷', R⁸', R¹", R²", R³", R⁴", R⁵", R⁶", R⁷", R⁸" are selected from:
-H, -(C₁-C₁₂)-alkyl, -O-(C₁-C₁₂)-alkyl, -O-(C₆-C₂₀)-aryl, -(C₆-C₂₀)-aryl, -S-alkyl, -S-aryl, halogen, COO-(C₁-C₁₂)-alkyl, CONH-(C₁-C₁₂)-alkyl, -CO-(C₁-C₁₂)-alkyl, -CO-(C₆-C₂₀)-aryl, -COOH, -OH, -SO₃H, -NH₂, -N[(C₁-C₁₂)-alkyl]₂;
R⁹', R¹⁰', R¹¹', R¹²', R⁹", R¹⁰", R¹¹", R¹²" are selected from:
-H, -(C₆-C₂₀)-aryl;
where the alkyl and aryl groups mentioned may be substituted as follows:
(C1-C12)-alkyl and O-(C1-C12)-alkyl may each be unsubstituted or substituted by one or more identical or different radicals selected from (C3-C12)-cycloalkyl, (C3-C12)-heterocycloalkyl, (C6-C20)-aryl, fluorine, chlorine, cyano, formyl, acyl and alkoxycarbonyl;
substituted -(C6-C20)-aryl groups and -(C6-C20)-aryl-(C6-C20)-aryl groups may have one or more substituents, depending on the ring size; these substituents are each independently selected from -H, -(C1-C12)-alkyl, -O-(C1-C12)-alkyl, -O-(C6-C20)-aryl, -(C6-C20)-aryl, halogen, -COO-(C1-C12)-alkyl, -CONH-(C1-C12)-alkyl, - (C6-C20)-aryl-CON[(C1-C12)-alkyl]2, -CO-(C1-C12)-alkyl, -CO-(C6-C20)-aryl, -COOH, -OH, -SO₃H, -SO3Na, NO2, - CN, -NH2, -N[(C1-C12)-alkyl]2.

2. Compound according to Claim 1,
where R¹, R², R³, R⁴, R⁵, R⁶, R⁷, R⁸ are selected from:
-H, -(C₁-C₁₂)-alkyl, -O-(C₁-C₁₂)-alkyl, -O-(C₆-C₂₀)-aryl, -S-alkyl, -S-aryl.

3. Compound according to either of Claims 1 and 2, where R¹, R², R³, R⁴, R⁵, R⁶, R⁷, R⁸ are selected from:
-H, -(C₁-C₁₂)-alkyl, -O-(C₁-C₁₂) -alkyl, -O-(C₆-C₂₀) -aryl .

4. Compound according to any of Claims 1 to 3,
where R⁴ and R⁵ are each -H.

5. Compound according to any of Claims 1 to 4,
where R¹', R²', R³', R⁴', R⁵', R⁶', R⁷', R⁸', R¹", R²" , R³", R⁴", R⁵", R⁶", R⁷", R⁸" are selected from:
-H, -(C₁-C₁₂)-alkyl, -O-(C₁-C₁₂)-alkyl, -O-(C₆-C₂₀)-aryl, -S-alkyl, -S-aryl.

6. Compound according to any of Claims 1 to 5,
where R¹', R²', R³', R⁴', R⁵', R⁶', R⁷', R⁸', R¹", R²", R³", R⁴", R⁵", R⁶", R⁷", R⁸" are selected from:
-H, -(C₁-C₁₂) -alkyl, -O-(C₁-C₁₂)-alkyl, -O-(C₆-C₂₀)-aryl.

7. Compound according to any of Claims 1 to 6,
where R¹', R²', R³', R⁴', R⁵', R⁶', R⁷', R⁸', R¹", R²", R³", R⁴", R⁵", R⁶", R⁷", R⁸" are each -H.

8. Compound according to any of Claims 1 to 7,
where R⁹', R¹⁰', R¹¹', R¹²', R⁹", R¹⁰", R¹¹", R¹²" are each -(C₆-C₂₀)-aryl.

9. Compound according to any of Claims 1 to 8,
where R⁹', R¹⁰'_{,} R¹¹', R¹²', R⁹", R¹⁰", R¹¹", R¹²" are each phenyl.

10. Compound according to any of Claims 1 to 9,
where R⁹', R¹⁰', R¹¹', R¹²', R⁹", R¹⁰", R¹¹", R¹²" are each the same radical.

11. Compound according to any of Claims 1 to 10,
where the compound has the general structure (I).

12. Compound according to any of Claims 1 to 10,
where the compound has the general structure (II).

13. Complex comprising:
- a compound according to any of Claims 1 to 12,
- a metal atom selected from: Rh, Ru, Co, Ir.

14. Use of a compound according to any of Claims 1 to 12
for catalysing a hydroformylation reaction.

15. Process comprising the following process steps:
a) initially charging an olefin,
b) adding a complex according to Claim 13,
or a compound according to any of Claims 1 to 12 and a substance having a metal atom selected from: Rh, Ru, Co, Ir,
c) feeding in H₂ and CO,
d) heating the reaction mixture, with conversion of the olefin to an aldehyde.

## Revendications

1. Composé, qui présente une des deux structures générales I ou II : 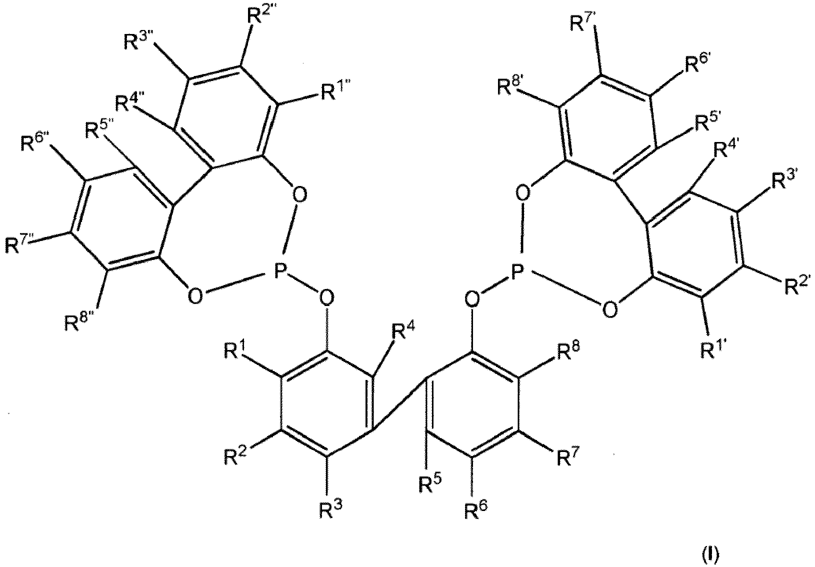 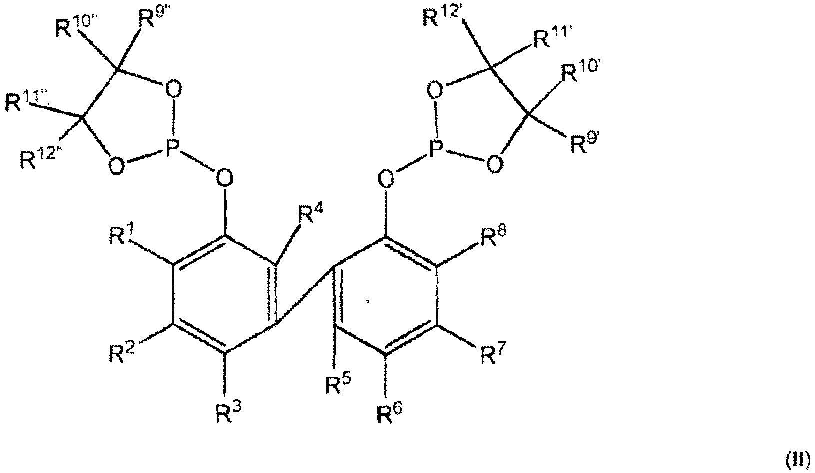 dans lesquelles
R¹, R², R³, R⁴, R⁵, R⁶, R⁷, R⁸ sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle, halogène, COO-alkyle en (C₁-C₁₂), CONH-alkyle en (C₁-C₁₂), -CO-alkyle en (C₁-C₁₂), -CO-aryle en (C₆-C₂₀), -COOH, -OH, -SO₃H, -CN, -NH₂, -N[alkyle en (C₁-C₁₂)]₂ ;
R¹', R²', R³', R⁴', R⁵', R⁶', R⁷', R⁸', R¹", R²", R³", R⁴", R⁵", R⁶", R⁷", R⁸" sont choisis parmi :
-H, -alkyle en (C₁-C₁₂), -O-alkyle en (C₁-C₁₂), -O-aryle en (C₆-C₂₀), -aryle en (C₆-C₂₀), -S-alkyle, -S-aryle, halogène, COO-alkyle en (C₁-C₁₂), CONH-alkyle en (C₁-C₁₂), -CO-alkyle en (C₁-C₁₂), -CO-aryle en (C₆-C₂₀), -COOH, -OH, -SO₃H, -NH₂, -N[alkyle en (C₁-C₁₂)]₂ ;
R⁹', R¹⁰', R¹¹', R¹²', R⁹", R¹⁰", R¹¹", R¹²" sont choisis parmi :
-H, -aryle en (C₆-C₂₀) ;
les groupes alkyle et aryle mentionnés pouvant être substitués de la manière suivante :
alkyle en (C₁-C₁₂) et O-alkyle en (C₁-C₁₂) peuvent chacun être non substitués ou substitués par un ou plusieurs radicaux identiques ou différents, qui sont choisis parmi cycloalkyle en (C₃-C₁₂), hétérocycloalkyle en (C₃-C₁₂), aryle en (C₆-C₂₀), fluor, chlore, cyano, formyle, acyle ou alcoxycarbonyle ;
les groupes aryle en (C₆-C₂₀) et les groupes aryle en (C₆-C₂₀)-aryle en (C₆-C₂₀) substitués peuvent comprendre, en fonction de la taille du cycle, un ou plusieurs substituants ; ces substituants sont choisis indépendamment les uns des autres parmi -H, -alkyle en (C₁-C₁₂), -O-alkyle en (C₁-C₁₂), -O-aryle en (C₆-C₂₀), - aryle en (C₆-C₂₀), -halogène, -COO-alkyle en (C₁-C₁₂), - CONH-alkyle en (C₁-C₁₂), -aryle en (C₆-C₂₀)-CON [alkyle en (C₁-C₁₂)]₂, -CO-alkyle (C₁-C₁₂), -CO-aryle en (C₆-C₂₀), - COOH, -OH, -SO₃H ; - SO₃Na, -NO₂, -CN, -NH₂, -N[alkyle en (C₁-C₁₂)]₂.

2. Composé selon la revendication 1, dans lequel R¹, R², R³, R⁴, R⁵, R⁶, R⁷, R⁸ sont choisis parmi :
-H, -alkyle en (C₁-C₁₂), -O-alkyle en (C₁-C₁₂), -O-aryle en (C₆-C₂₀), -S-alkyle, -S-aryle.

3. Composé selon l'une quelconque des revendications 1 ou 2, dans lequel R¹, R², R³, R⁴, R⁵, R⁶, R⁷, R⁸ sont choisis parmi :
-H, -alkyle en (C₁-C₁₂), -O-alkyle en (C₁-C₁₂), -O-aryle en (C₆-C₂₀).

4. Composé selon l'une quelconque des revendications 1 à 3, dans lequel R⁴ et R⁵ représentent -H.

5. Composé selon l'une quelconque des revendications 1 à 4, dans lequel R¹', R²', R³', R⁴', R⁵', R⁶', R⁷', R⁸', R¹", R²", R³", R⁴", R⁵", R⁶", R⁷", R⁸" sont choisis parmi :
-H, -alkyle en (C₁-C₁₂), -O-alkyle en (C₁-C₁₂), -O-aryle en (C₆-C₂₀), -S-alkyle, -S-aryle.

6. Composé selon l'une quelconque des revendications 1 à 5, dans lequel R¹', R²', R³', R⁴', R⁵', R⁶', R⁷', R⁸', R¹", R²", R³", R⁴", R⁵", R⁶", R⁷", R⁸" sont choisis parmi :
-H, -alkyle en (C₁-C₁₂), -O-alkyle en (C₁-C₁₂), -O-aryle en (C₆-C₂₀).

7. Composé selon l'une quelconque des revendications 1 à 6, dans lequel R¹', R²', R³', R⁴', R⁵', R⁶', R⁷', R⁸', R¹", R²", R³", R⁴", R⁵", R⁶", R⁷", R⁸" représentent -H.

8. Composé selon l'une quelconque des revendications 1 à 7, dans lequel R⁹', R¹⁰', R¹¹', R¹²', R⁹", R¹⁰", R¹¹", R¹²" représentent -aryle en (C₆-C₂₀).

9. Composé selon l'une quelconque des revendications 1 à 8, dans lequel R⁹', R¹⁰', R¹¹', R¹²', R⁹", R¹⁰", R¹¹", R¹²" représentent phényle.

10. Composé selon l'une quelconque des revendications 1 à 9, dans lequel R⁹', R¹⁰', R¹¹', R¹²', R⁹", R¹⁰", R¹¹", R¹²" représentent le même radical.

11. Composé selon l'une quelconque des revendications 1 à 10, dans lequel le composé présente la structure générale (I).

12. Composé selon l'une quelconque des revendications 1 à 10, dans lequel le composé présente la structure générale (II).

13. Complexe, comprenant :
- un composé selon l'une quelconque des revendications 1 à 12,
- un atome métallique choisi parmi : Rh, Ru, Co, Ir.

14. Utilisation d'un composé selon l'une quelconque des revendications 1 à 12 pour la catalyse d'une réaction d'hydroformylation.

15. Procédé comprenant les étapes de procédé suivantes :
a) le chargement d'une oléfine,
b) l'ajout d'un complexe selon la revendication 13,
ou d'un composé selon l'une quelconque des revendications 1 à 12 et d'une substance qui comprend un atome métallique choisi parmi : Rh, Ru, Co, Ir,
c) l'introduction d'H₂ et de CO,
d) le chauffage du mélange réactionnel, l'oléfine étant transformée en un aldéhyde.
